# EUROPEAN PATENT SPECIFICATION

(11) **EP 2 590 615 B1**
(45) Date of publication and mention of the grant of the patent: **26.08.2015**
(21) Application number: 11730324.8
(22) Date of filing: 08.07.2011
(51) Int. Cl.: A61J 3/10, A61J 3/00, B30B 5/06, B30B 11/34, A61K 9/20

(54) **Multi-layer tablet formation by adhering tablet bodies together**
Herstellung einer mehrschichtigen Tablette mittels Zusammenhaftung von Tablettenkörpern
Formation de comprimés multicouches par collage ensemble de corps de comprimé

(30) Priority: 09.07.2010 EP 10169097
(43) Date of publication of application: 15.05.2013
(73) Proprietor: Research Center Pharmaceutical Engineering GmbH, 8010 Graz (AT)
(72) Inventor: KHINAST, Johannes, A-8010 Graz (AT); KLEIN, Thomas, A-8652 Kindberg (AT); SIRNIK, Bernhard, A-8642 St. Lorenzen im Mürztal (AT); HOFER, Johannes, A-8010 Graz (AT); REDLINGER-POHN, Jakob, A-8010 Graz (AT); GRUBER, Michael, A-8010 Graz (AT)
(74) Representative: Dilg, Haeusler, Schindelmann Patentanwaltsgesellschaft mbH
(86) International application number: PCT/EP2011/061705
(87) International publication number: WO 2012/004408

(56) References cited:
- EP-A1- 1 362 582
- DE-A1- 19 539 359
- US-A1- 2003 059 614

## Description

This application claims the benefit of the filing date of EP 10169097.2 filed 9 July 2010.

The invention relates to an apparatus for manufacturing a multi-layer tablet.

Moreover, the invention relates to a method of manufacturing a multi-layer tablet.

Furthermore, the invention relates to a multi-layer tablet.

A tablet is a pharmaceutical dosage form which comprises a mixture of active substances and excipients, usually in powder form, compressed or compacted into a solid. The excipients can include diluents, binders or granulating agents, glidants (flow aids) and lubricants to ensure efficient tabletting. Disintegrants may promote tablet break-up in the digestive tract. Sweeteners or flavors may enhance taste. Pigments may make the tablets visually attractive. A polymer coating may be applied to make the tablet smoother and easier to swallow, to control the release rate of the active ingredient, to make it more resistant to the environment (extending its shelf life), or to enhance the tablet's appearance.

Multi-layer tablets may comprise multiple active substances and excipients. Different powders having different active substances may be pressed together to form a tablet. Multi layer tablets are hence made by compression of two or more layers of granules or powders on top of the other. Due to exposed edges of the layers, multi layer tablets may have the appearance of a sandwich. Common rotary presses usually allow for the compression of two or three layers. However, the compression of more layers can be accomplished.

Vogeleer, Jan, "Bi-layer tablets--why special technology is required: the courtoy-R292F tablet press, designed for quality bi-layer tablets. (Application Area)", Pharmaceutical Processing , September 1, 2002, gives an overview over the technology of multi layer tablets.

Multi-layer tablets have certain advantages: Incompatible substances can be merged into one dosage form. If the small area of contact between two layers is still sufficient to result in significant and adverse interaction between the substances of the layers, a third layer to separate these materials can be introduced. Multi-layer tablets can be designed to achieve sustained release of one drug molecule. One layer carries the dose for immediate release, while the other causing the prolonged drug-release effect. Processing layers of different colours result in easy identification of the product. The weight of each layer can be controlled more easily than in compression-coated tablets. In comparison to compression-coated tablets also less materials are required resulting in thinner tablets of less weight. Additionally off-centering as seen for compression coats and inlay tablets does not occur in the production of layered tablets.

Conventionally, layers are compressed by the same punches in the same machine. However, there are limitations of the conventional production method: The layer thickness is limited by the capacity of the tablet press and the number of layers. Furthermore the thickness of a single layer is dependent on the fineness of the granulation or the size of the powder particles. Generally, the tablet size cannot exceed that of single layer compacts, depending on the capacity of the tableting equipment. Fines of a size below an average size of 150 microns should be avoided to provide a sharp resolution between the two layers. Large particles or granules of a size over 1,0 mm need to be avoided to provide a clean scrape off before compaction. Materials that smear or coat on the die table should be avoided to provide uncontaminated layers. Low moisture is essential if incompatible materials are used. Weak granules that break down easily should be avoided to achieve high layer resolution. Bonding strength between the layers should be insured. Therefore excessive use of lubricants should be avoided to achieve appropriate adhesion of the layers. Especially metallic stearates, like Mg Stearate can cause problems because they interfere with bonding mechanisms. Generally, the bonding of adjacent layers is crucial. Separation of the two layers can occur several days after compression if the bonding was too poor.

US 2009/0155315 A1 discloses a pharmaceutical and pharmaceutical-like product which provides a plurality of components having active agents that are delivered in a single delivery entity or vehicle. The product allows for selective control of the release rates of each of the active agents while still being delivered in a single product.

US 2003/0070903 A1 discloses systems, methods and apparatuses for manufacturing dosage forms, and dosage forms made using such systems, methods and apparatuses. Compression, thermal cycle molding, and thermal setting molding modules are disclosed. One or more of such modules may be linked, preferably via a transfer device, into an overall system for making dosage forms.

WO 2007/062323 A1 discloses a method and an apparatus for assembling a plurality of independently formed solid components thereby forming a single delivery vehicle for a pharmaceutical or pharmaceutical-like product. The solid components can be held and fed to the apparatus via a plurality of magazines. Pusher rods and the like can be used for positioning each of the solid components. Where the components are connected via a bonding liquid, a sprayer is provided and compression pins or the like press the components with the bonding liquid together to form the final product. A rivet or other connection structure can also be used and driven through holes in each of the solid components to form the final product.

It is an object of the invention to provide an efficient way of manufacturing multi-layer tablets.

In order to achieve the object defined above, apparatuses for manufacturing a multi-layer tablet, a method of manufacturing a multi-layer tablet, and a tablet according to the independent claims are provided.

According to an exemplary embodiment of the invention, an apparatus for manufacturing a multi-layer tablet is provided, the apparatus comprising a supply unit adapted for providing a first tablet body comprising at least a compressed or compacted first powder having one or more first pharmacologically active substances, and adapted for providing a second tablet body comprising at least a compressed or compacted second powder (which may be the same powder as the first powder or which may be another powder than the first powder) having one or more second pharmacologically active substances (which may be the same pharmacologically active substance as the first pharmacologically active substance or which may be another pharmacologically active substance than the first pharmacologically active substance), and an adhering unit for forming the multi-layer tablet by adhering the preformed first tablet body and the preformed second tablet body together by an adhesive.

According to another exemplary embodiment of the invention, a method of manufacturing a multi-layer tablet is provided. The method comprises providing a first tablet body comprising a compressed first powder having a first pharmacologically active substance, providing a second tablet body comprising a compressed second powder having a second pharmacologically active substance, and forming the multi-layer tablet by adhering the first tablet body and the second tablet body together by an adhesive.

According to still another exemplary embodiment of the invention, a multi-layer tablet is provided which comprises a first tablet body comprising a compressed first powder having a first pharmacologically active substance, a second tablet body comprising a compressed second powder having a second pharmacologically active substance, an adhesive adhering the first tablet body and the second tablet body together. The multi-layer tablet may be formed by the above apparatus and/or by the above method.

In the context of this application, the term "tablet" may particularly denote a solid-state body formed based on a powder material and further ingredient(s) by pressing. A tablet can be a three-dimensional body having an extension in any of the three coordinate axes between 1 mm and 50 mm, particularly between 3 mm and 2 cm. A tablet may comprise powder as matrix material, one or more pharmacologically active substances as active agent, and optionally one or more additional excipients such as antiadherents, binders, coatings, disintegrants, flavors, colors, lubricants, glidants, preservatives, sorbents, etc.

In the context of this application, the term "multi-layer tablet" may particularly denote a tablet comprising two or more physically distinguishable layers connected to one another by a biocompatible glue. Different layers or structures may comprise the same of different physiologically active substances in the same or different quantities or concentrations, the same of different powder or composition of powders, and/or optionally the same or different further expedients. The term "multi-layer" should not be understood in a strict sense and does not necessarily be composed of strictly planar layers. It covers any tablet shape having two or more physically distinguishable portions formed before being connected to one another by an adhesive and constituting the multiple layers. The term "multi-layer" particularly covers bi-layer tablets, triple-layer tablets, four-layer tablets, etc.

In the context of this application, the term "powder" may particularly denote small solid particles such as granulates, which may comprise residual moisture. A powder may be a solid substance in the form of tiny lose particles, i.e. pulverized material, in contrast to a tablet which is a solid-state body formed of a large plurality of such powder particles by compressing or compacting. The dimension of the particles of the powder may be in a range between 100 nm and 2000 µm, particularly in a range between 1 µm and 1000 µm. The term "powder" may particularly denote any substance which serves as a carrier matrix for a pharmacologically active substance in a tablet. Hence, in one embodiment, the powder may be a substance being separate from the pharmacologically active substance. However, since some pharmacologically active substances can be directly pressed without an additional matrix, the term "powder" may cover also any substance which is a pharmacologically active substance itself. Hence, in another embodiment, the powder may be a substance which is the pharmacologically active substance itself. A powder is a dry material composed of a large number of very fine particles that may flow freely when shaken or tilted. Powders refer to granular materials that have fine grain sizes, and that therefore have some tendency to form clumps when flowing. The powder may serve as excipient of the tablet and may be an inactive substance used as a carrier for the pharmacologically active substance of a medication. The pharmacologically active substance may be dissolved into or mixed with an excipient such as the powder. Excipients may be used to bulk up formulations that contain very potent active ingredients, to allow for convenient and accurate dosage. In addition to their use in the single-dosage quantity, excipients can be used in the manufacturing process to aid in the handling of the active substance concerned.

In the context of this application, the term "adhesive" may particularly denote a biocompatible (particularly compatible to be administered to a human being in quantities of a tablet) material which is capable of connecting tablet bodies made of compressed powder. Such an adhesive or biocompatible glue may be in a liquid or semiliquid or solid-state that adheres or bonds tablet bodies together. Adhesives may come from natural and/or synthetic sources. Alternatively, the adhesive may be a biocompatible adhesive tape.

The term "pharmacologically active substance" may particularly denote any substance which has a therapeutic effect on a physiological subject such as a human being or an animal. It may include substances forming the functional basis for medicaments, and also includes the field of homoeopathic applications.

The term "tablet body" may particularly denote a preformed tablet layer in solid form, i.e. formed based on compressed powder. The tablet body may be considered as a semifinished product to be further processed by combining it with at least one further tablet body to finally form a tablet. However, such a tablet body may already have the appearance of a tablet.

In the context of this application, the term "supply unit" may particularly denote a functional section of the apparatus which serves for supplying at least two tablet bodies to a target position for subsequent adhering to one another. The supply unit may be formed by one or multiple components.

In the context of this application, the term "adhering unit" may particularly denote a functional section of the apparatus which serves for adhering at least two tablet bodies to one another by an adhesive material. The adhering unit may also provide the adhesive to a target position in a desired quantity and may be formed by one or multiple components.

According to an exemplary embodiment, a multiple component tablet may be manufactured by firstly providing at least two tablet bodies which are already in a compacted, i.e. no more pulverized, form. An adhesive material is then applied to at least one of the tablet bodies to connect the tablet bodies to form the actual tablet. Fall tests, pull tests and torsion experiments have confirmed that, upon applying mechanical stress to a tablet formed in such a manner, the place of fracture of the multi-layer tablet is generally spatially apart from the adhesion position, so that it is presently believed that the robustness of the tablet is even stronger close to the adhesion portion as compared to other portions.

In a first preferred aspect, the supply unit comprises a first conveyor belt adapted for conveying the first tablet body to an adhering position and comprises a second conveyor belt adapted for conveying the second tablet body to the adhering position, wherein the first conveyor belt and the second conveyor belt are arranged to spatially converge at the adhering position so that the first tablet body and the second tablet body are pressed together by the converging first conveyor belt and second conveyor belt at the adhering position. Such a tandem arrangement of conveyor belts engaging the tablet bodies allows for an accurate and reliable connection between the tablet bodies and allows for a high throughput while being compact in construction.

In a second preferred aspect, a rotatable wheel having a plurality of accommodation spaces along a circumference of the wheel is provided, wherein the supply unit is positioned relative to the wheel for supplying the first tablet body and the second tablet body to one of the plurality of accommodation spaces. Such a rotating wheel arrangement with circumferential tablet body accommodation spaces allows for an accurate and reliable connection between the tablet bodies and allows for a high throughput while being compact in construction.

In a third preferred aspect, a press mechanism is provided which is adapted for applying a predefined pressure to the first tablet body, the second tablet body and the adhesive arranged between the first tablet body and the second tablet body at an adhering position, wherein the press mechanism comprises a movable element (or actuator) such as a movable piston, wherein a channel is formed within the movable element for supplying the adhesive. Such a for instance reciprocating element with integral adhesive supply channel allows for an accurate and reliable connection between the tablet bodies and allows for a high throughput while being compact in construction.

In the following, further exemplary embodiments of the apparatus will be explained. However, these embodiments also apply to the method and to the tablet.

Various methods of applying the adhesive can be carried out in exemplary embodiments of the invention, such as dipping, spaying, brushing, dispensing, printing, calender coating, film coating, etc.

In an embodiment, the apparatus may comprise a compressing unit adapted for compressing the first powder comprising the first pharmacologically active substance to form the first tablet body. The compressing unit may be further adapted for independently compressing the second powder comprising a second pharmacologically active substance to form the second tablet body. The compressing may be performed prior to the adhering. The compressing unit may be adapted for forwarding the first tablet body and the second tablet body to the supply unit. Thus, applying a physical pressure on the powders with the respective pharmacologically active substances may allow to manufacture the two tablet bodies separately from one another. Experiments have shown that the separate compressing of the tablet body before connecting these individual tablet bodies by applying an adhesive allows to obtain a very robust tablet.

The adhering unit may comprise an adhesive metering unit adapted for providing the adhesive with a predefined application rate to at least one of the tablet bodies. In an embodiment, the adhesive metering unit may comprise a microdosing unit adapted for providing a microdose (particularly a dose with a volume in the order of magnitude of microliters, for instance - but not limited to - between 0,1 µl to 1000 µl of the adhesive to the adhering unit. It has turned out that, depending on the material of the powder and the pharmacologically substances, depending on the number of tablet bodies to be connected, and depending on the shape of the tablet bodies, the amount of adhesive to be applied for a reliable connection has to be adjusted very accurately by the microdosing unit.

In an embodiment, the adhesive metering unit may be piezo-controlled. By selectively activating or deactivating a piezo element acting on a dispenser capillary or the like may allow to properly dose the adhesive. In another embodiment, the adhesive metering unit may be an adhesive metering valve which can be controlled to be open for supplying adhesive or closed for disabling supply of adhesive.

Furthermore, the adhesive metering unit may comprise a temperature adjustment unit adapted to control a temperature of the adhesive before ejection of the adhesive through an orifice. Such a temperature adjustment unit may particularly be a heat application unit adapted to apply heat to the adhesive before ejection of the adhesive through the orifice. Therefore, it is possible to precisely control the viscosity of the adhesive before ejection, thereby also allowing to properly adjust the fluid flow properties before (and after) ejection. In an embodiment, it may also be possible that also cooling of the adhesive is enabled, for instance in a scenario in which the temperature of the adhesive is too high for a proper ejection. In this scenario, a cooling unit such as a Peltier element can be used as the tempering unit. Such a Peltier element may also be used for a heating. In a scenario, in which only heating is required, an ohmic heating element may be provided such as a coil spirally wound around a capillary of the adhesive metering unit and/or around an optional fluidic conduit between the capillary and an adhesive container. Additionally or alternatively, an ohmic heating element may be provided for heating the adhesive within the container (such as a coil spirally wound around the adhesive container). Such a temperature adjustment unit, particularly heating element, may also be arranged within the capillary and/or the conduit.

In an embodiment, the temperature adjustment unit, particularly the heat application unit, may be adapted to apply heat to the adhesive before leaving the capillary. Therefore, already in a state in which the adhesive is properly controllable, i.e. within the capillary, the temperature adjustment may be performed. Then, also the viscosity properties which are of relevance with regard to the fluid properties when leaving the capillary can be properly controlled. By pre-heating the adhesive, the viscosity may be reduced and the surface tension may be reduced to achieve constant properties of the adhesive and to facilitate or enable printing of the adhesive, which may be solid at room temperature. Furthermore by pre-heating a faster drying of the adhesive may be achieved. This may increase the speed of manufacturing the tablets.

In an embodiment, the apparatus may comprise a temperature adjustment unit adapted for adjusting a temperature of the first tablet body, the second tablet body and/or the adhesive. Particularly, the temperature adjustment unit may be arranged at the adhering unit. By taking this measure, it is possible to precisely adjust the temperature during the adhesion process. It is possible to promote a proper adhesion between the tablet bodies if the temperature is increased during the adhesion procedure. For instance, a solid adhesive such as wax may be liquefied temporarily by such a temperature increase. After having connected the tablet bodies by the adhesive material, it has turned out to be advantageous to cool the tablet or portions thereof, thereby promoting a fast and reliable connection between the different components of the tablet. By heating the adhesive only before or during the adhesion procedure and subsequent cooling it, it is also possible to prevent solvent (for instance water) in the core of the tablet bodies from evaporating. Furthermore, it may be advantageous that the adhesion rapidly dries so that the robustness of the multilayer tablet is already given at the time of ejecting the manufactured tablet from the manufacturing device.

In an embodiment, the adhesive and/or the one or more of the tablet bodies is/are heated to a temperature in a range between 25 °C and 150 °C, particularly in a range between 40 °C and 80 °C, more particularly in a range between 50 °C and 70 °C, to promote the adhering procedure. This may guarantee a fast adhering process due to the thermal energy which may accelerate the adhering procedure. Furthermore, such a heating may also liquefy an adhesive (such as a wax) being solid at room temperature during the adhesion process. At the same time, the given temperature range may ensure that physiologically active substances in the tablet bodies are not deteriorated or rendered physiologically inactive by excessive heat.

After the adhering, the multi-layer tablet may be cooled to a lower temperature, for instance to or below room temperature. An appropriate cooling temperature may be in a range between 0 °C and 30 °C, particularly in a range between 10 °C and 30 °C for a rapid solidifying of the adhesion material after the adhesion procedure, thereby increasing the throughput of manufactured multi-layer tablets.

In an embodiment, the apparatus may comprise a press mechanism adapted for applying a predefined pressure to the first tablet body, the second tablet body and the adhesive arranged between the first tablet body and the second tablet body at the adhering position. The pressure force applied by the press mechanism in a range between 1 N and 500 N, particularly in a range between 10 N and 50 N. Such a press mechanism may be a physical unit capable of applying an overpressure to the multi-layer tablet during its formation to increase the robustness of the manufactured tablet.

According to an exemplary embodiment, the supply unit may comprise a tablet body retention mechanism adapted for retaining tablet bodies at a later position in a queue of tablet bodies from being supplied from the supply unit to the adhering unit before tablet bodies at an earlier position in the queue have left the adhering unit. Such a retention mechanism may be a physical structure which is capable of applying a predefined holding force to tablet bodies which are not yet to be processed to avoid that a too large number of tablet bodies enter the adhering unit for being adhered to one another by the adhesive. By such a tablet body retention mechanism, it is possible to reliably control the replenishment of additional tablet bodies. Thus, the retention mechanism may safely prevent failure of the manufacturing apparatus due to clogging particularly within the adhering unit.

According to an exemplary embodiment, the tablet body retention mechanism may comprise a retention spring (or alternatively a plurality of retention springs) which may be arranged at the supply unit for exerting a retention force to tablet bodies at the later position inhibiting the tablet bodies at the later position from advancing to the adhering unit before the tablet bodies at the earlier position have left the adhering unit. Such a retention spring arrangement may be advantageous for a large scale manufacture of tablets. The retention springs may be flat springs (leaf springs), coil springs, etc. It may be particularly advantageous that a leaf spring is arranged in a channel of the supply unit through which the tablet bodies are conveyed. Hence, at a certain position, a too early fresh supply of the tablet bodies may be inhibited by the force exerted by the retention springs. The exertion force of the retention springs may be configured in such a manner that after having transported away earlier processed tablet bodies which are already connected to a multi-layer tablet, the feed force (for instance the gravitational force of a queue of subsequent tablet bodies) may be larger than the retention force of the retention spring. This may allow to properly control the speed of processing the tablet bodies for manufacturing tablets.

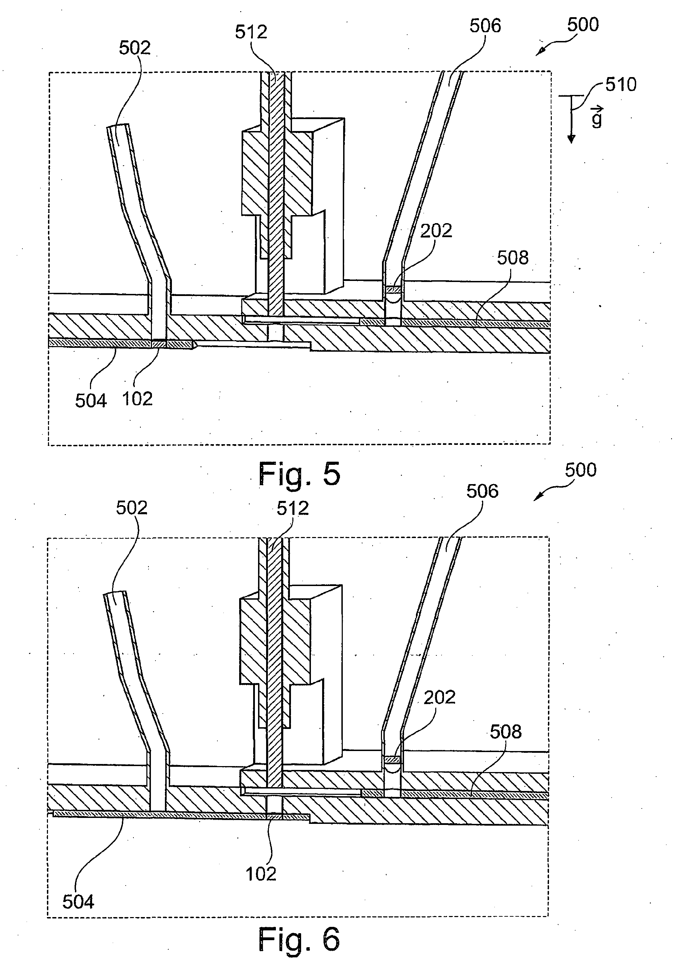

According to an embodiment (compare Fig. 5 to Fig. 10), the supply unit may comprise a first supply channel and a first feeder mechanism adapted for conveying the first tablet body towards an adhering position at the adhering unit. The term "adhering position" may denote a portion or section of the apparatus at which the tablet bodies and the adhesive are brought in interaction with one another to trigger their connection. Hence, the adhering position may be denoted as the position at which the actual adhesion between the two or more semifinished tablet bodies by means of the adhesive takes place. In an embodiment, the first supply channel may be a supply tube having a lumen through which the first tablet body (or a queue of multiple first tablet bodies) may be moved. In an alternative embodiment, the first supply channel may be a channel formed within a solid-state material such as a plate. The first feeder mechanism may be a mechanism having a movable part which is capable of moving coordinated with the supply of the first tablet body by first supply channel so as to transport the first tablet body supplied through the first supply channel to a desired destination, particularly to the adhering position.

In one embodiment, the supply unit may additionally comprise a second supply channel and a second feeder mechanism adapted for conveying the second tablet body (or a queue of multiple second tablet bodies) towards the adhering position at the adhering unit. In such an embodiment, an additional supply channel and an additional feeder mechanism is provided for the second tablet body which may be configured in a similar or identical manner as the above-mentioned first supply channel and first feeder mechanism. Thus, with a similar mechanism as the first tablet body, also the second tablet body may be moved towards the adhering position for the subsequent adhesion procedure. The provision of separate supply channels and feeder mechanisms for the different tablet bodies has the advantage of a fast manufacture of the tablets, since the first supply channel and the first feeder mechanism on the one hand and the second supply channel and the second feeder mechanism on the other hand may be operated simultaneously.

In an alternative embodiment, the first supply channel and the first feeder mechanism may be adapted for also conveying the second tablet body (or a queue of multiple second tablet bodies) towards the adhering position at the adhering unit. In such an embodiment, supply of first and second tablet bodies may be performed by the same supply channel so that an additional supply channel or an additional feeder mechanism may be omitted, which results in a compact architecture.

In both alternatives, it is possible that the supply of tablet bodies via the one or more supply channels is driven at least partially by a gravitational force exerted on the tablet bodies as a consequence of the spatial arrangement of the supply channel(s). This may be achieved by arranging the supply channel(s) for instance along a vertical or slanted direction in such a manner that at least a component of the gravitational force conveys the tablet body automatically towards the respective feeder mechanism. Therefore, the hardware effort and the energy consumption needed for feeding the tablet bodies is very small.

Still referring to the previously described embodiment, the first feeder mechanism and, if any, the second feeder mechanism may be arranged for horizontally sliding the respective tablet body from an end position of the at least one supply channel towards the adhering position at the adhering unit. The tablet bodies may fall through the supply channel(s) towards a stop position and may be shifted from there by a lateral movement of the respective feeder towards the adhering position. Hence, lateral sliders may be provided as the feeder mechanism configured to shift the respective tablet body to a destination at which the adhesive connection takes place.

In the previously described embodiment, the press mechanism may comprise a movable mechanism such as a movable piston, for instance a reciprocatable piston (which may move upwardly and downwardly), which may be adapted for applying the predefined pressure along a compressing direction which is angularly arranged relative to the horizontal sliding direction of the first feeder mechanism and, if any, of the second feeder mechanism. For instance, the motion direction of the element (for instance piston) may be perpendicular or at least basically perpendicular relative to the horizontal sliding direction of the feeder(s). In such a scenario, the supply of tablet bodies occurs perpendicularly to the pressing direction.

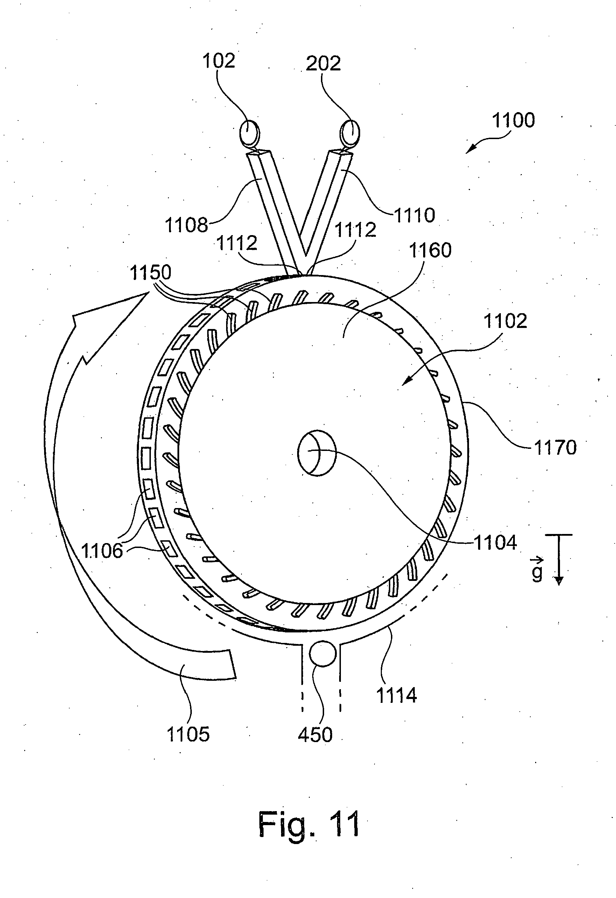

According to another embodiment (compare Fig. 11 to Fig. 14), the apparatus may comprise a rotatable wheel which has a plurality of accommodation spaces (or accommodation chambers or accommodation recesses) along a circumference of the wheel. The supply unit may be coupled to the wheel for supplying the first tablet body and the second tablet body (conveyed via the supply unit) to a respective one of the plurality of accommodation spaces. In such an embodiment, the rotatable wheel may act as a turret. Such a turret may be considered as a revolving structure having circumferentially distributed the plurality of accommodation spaces each capable of accommodating a tablet and having optionally provisions for producing this tablet partially within the respective accommodation space based on at least two tablet bodies and adhesive material. Such an architecture may be particularly appropriate for mass production of multi-layer tablets and also allows cooling of the readily manufactured tablet directly after adhesion by mere rotation of the wheel. During the motion of the wheel which transports off a certain tablet from a processing position, cooling by convection may take place. In such a turret configuration, the supply unit may be brought to a position relative to the turret so that the tablet body may fall into a respective one of the accommodation spaces to be automatically guided from the supply unit to the rotatable wheel. During this supply procedure, it is also possible to add the adhesive material to one, a part or all tablet bodies forming a multi-layer tablet. This mixture may be inserted in one of the accommodation spaces where a compression and/or an application of heat may take place for the connection procedure.

In the previously described embodiment, the supply unit may comprise a first supply channel adapted for conveying the first tablet body towards the one of the plurality of accommodation spaces. In one embodiment, the first supply channel may comprise a supply channel through which the respective tablet body may be transported. In an alternative integrated configuration, it is possible to configure the first supply channel as a through-hole in a plate or the like.

Still referring to the above embodiment, the supply unit may comprise a second supply channel adapted for conveying the second tablet body towards the one of the plurality of accommodation spaces. Hence, at a position where the two supply channels are closest to the turret, their lumens may approach one another or may converge. In case of more than two tablet bodies to be connected to a tablet, one or more additional supply channel may be possible as well. Providing separate supply channels for different tablet bodies may allow to manufacture the tablets with a high throughput.

Alternatively, the first supply channel described above may also be adapted for conveying the second (and if necessary a third, a fourth, etc.) tablet body towards the one of the plurality of accommodation spaces. Thus, it is also possible that the first supply channel supplies a part or all of the tablet bodies towards the turret. This may result in a very compact structure.

In an embodiment, the supply unit may be adapted as a stator remaining stationary fixed. In contrast to this, the wheel may be operated to rotate to thereby bring successively different ones of the plurality of accommodation spaces in alignment with the supply unit for enabling supply of tablet bodies to a corresponding one of the accommodation spaces being presently in alignment with the supply unit. It may be particularly appropriate to insert the tablet bodies into a respective accommodation space of the wheel at an uppermost position of the wheel, particularly when the wheel is arranged with the wheel axis perpendicular to the gravitational force. In such a scenario, the gravitational force will support the tablet bodies to be inserted into the accommodation spaces without an additional drive mechanism. Queues of tablet bodies may then be filled in the one or more supply channels so that, in each accommodation space being in present alignment with the outlets of the one or more supply channels, a defined number of tablet bodies is always inserted into the respective accommodation space. By rotating the wheel with the accommodation spaces, the queue of tablet bodies in the one or more supply channels move forward and automatically insert new tablet bodies in a new still empty accommodation space being presently in alignment with the one or more supply channels.

In an embodiment, the adhering unit may comprise an adhesive metering unit arranged for supplying the adhesive material to at least one of the two or more tablet bodies at a position in the supply unit which is directly upstream of a boundary between the supply unit and a respective one of the plurality of accommodation spaces. Therefore, adhesive material may be applied particularly to one of the tablet bodies immediately before the respective tablet body leaves the supply channel and enters an accommodation space in the wheel. Therefore, undesired adhesion between tablet bodies within the supply channels may be safely prevented. With the use of an adhesive metering unit, an amount of adhesive to be applied to a certain tablet body may be precisely controlled. For instance, a droplet of a certain volume may be applied to each tablet body in at least one supply channel. Adhesion application centrally in the supply channel has the advantage that it is not necessary to provide for an adhesion supply provision in each accommodation space.

In an embodiment, it is possible to apply adhesive not only to one, but to both (or to more than two) tablet bodies to be connected to one another before the connection process. This can be performed by one common adhesive supply unit (such as a metering unit) or by two (or more than two) adhesive supply units (such as metering units), each provided separately for a corresponding tablet body.

According to an embodiment, the above-mentioned press mechanism may, in the context of the turret embodiment, be arranged in at least a part of the plurality of accommodation spaces. For example, two opposing abutment structures may be arranged in specific ones or in all of the accommodation spaces. Upon moving one of these abutment structures relative to the other one, or upon moving both abutment structures towards one another, a compression force may be exerted on tablet bodies arranged within the corresponding accommodation space.

Still referring to the previously described embodiment, it is alternatively (to the application of the adhesive in the supply channels) also possible that the adhesive is applied to the tablet bodies within the accommodation space. In such an embodiment, the adhesive material is applied directly before connecting the tablet bodies to one another. This allows for a precise control of the adhesion procedure and prevents undesired adhesion between other components of the apparatus.

In an embodiment, the apparatus may comprise a tablet ejection unit adapted for ejecting a tablet from a respective one of the accommodation spaces after rotation about a predefined rotation angle with the wheel. Hence, when the wheel rotates, it may be desired that the insertion of the tablet bodies into the accommodation spaces takes place at the uppermost position of the wheel, because in this position there is no danger that the inserted tablet bodies fall out of the accommodation space due to the effect of gravitation. It is also possible to use the gravitational force acting on the tablet bodies for an automatic ejection of the manufactured tablet out of the respective accommodation space after certain angle of rotation. It may be particularly appropriate that the tablets leave the turret after a 180° rotation. After such a rotation angle, the tablets will automatically fall out of the turret, for instance in a tablet container or the like so that the emptied accommodation space is ready for a next production of a multi-layer tablet.

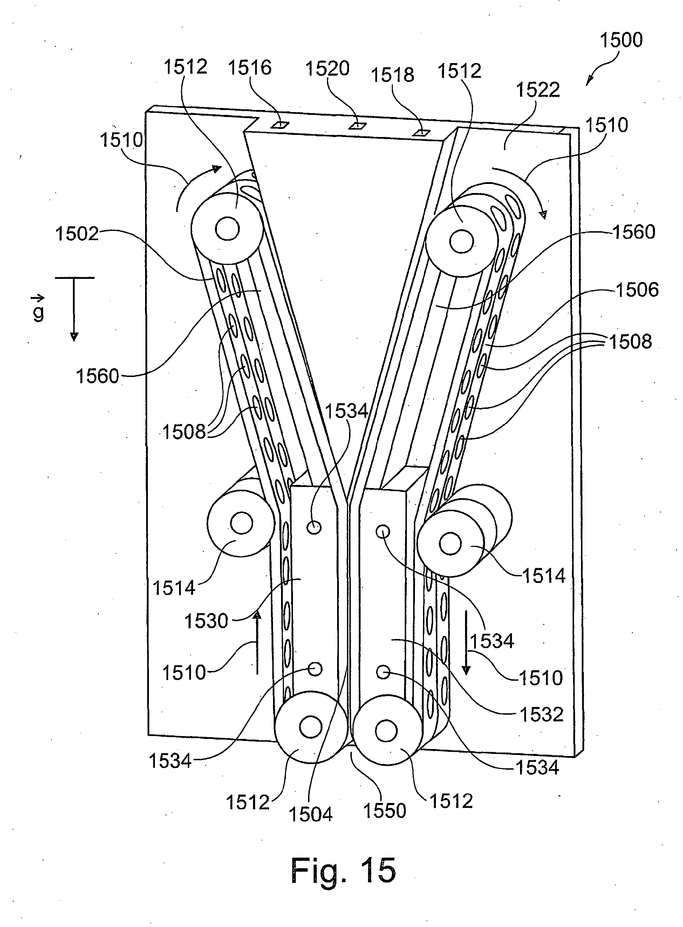

In an alternative embodiment (compare Fig. 15 to Fig. 16), the supply unit may comprise a first conveyor belt adapted for conveying the first tablet body to an adhering position and may comprise a second conveyor belt adapted for conveying the second tablet body to the adhering position. The first conveyor belt and the second conveyor belt may be arranged to spatially converge (i.e. to approach one another) at the adhering position so that the first tablet body and the second tablet body are pressed together by the converging first conveyor belt and second conveyor belt at the adhering position. Thus, two conveyor systems being arranged as cooperating endless conveyor structures may be used which is also advantageous in the view of a large scale production of multi-layer tablets. In such a rotating system, both belts transport respective tablet bodies to a position at which they are merged due to the spatially converging arrangement of the conveyor belts. At this converging position, the two or more tablet bodies are connected to one another by the influence of the adhesive promoted by the pressure applied due to the conversion. In other words, the tablet bodies are sandwiched between the two conveyor belts at their closest position relative to one another. The provision of the adhesive may be directly at the converging position or alternatively at a position upstream of the transportation path of one or both of the tablet bodies.

Still referring to the previously described embodiment, the first conveyor belt and/or the second conveyor belt may comprise a plurality of accommodation spaces arranged along a motion trajectory of the respective conveyor belt, each being adapted for accommodating a respective tablet body. The accommodation spaces of the respective conveyor belts may be arranged so that in the converging section, tablet bodies in opposing accommodation spaces of the two conveyor belts contact each other at a main surface of the tablet bodies. For instance, recesses may be formed in the conveyor belts at one continuous surface thereof. Each of the recesses may be shaped and dimensioned to be capable of receiving a respective one of the tablet bodies. If desired, a holding structure for a tablet body may be arranged within each of the accommodation spaces, such as a spring, a retractable engagement member, etc.

In an embodiment, at least one of the first conveyor belt and the second conveyor belt may comprise at least one drive roll adapted for driving, i.e. moving, the respective conveyor belt. Hence, the conveyor belt may be guided along the one or more drive rolls, wherein each drive roll may be moved by a drive such as a motor, wherein the respective first conveyor belt is also brought into motion due to a frictional force between the respective conveyor belt and the drive roll.

Still referring to the previously described embodiment, at least one of the first conveyor belt and the second conveyor belt may comprise at least one shaping roll adapted for defining a shape of the respective conveyor belt. Such a conveyor roll may be arranged inside or outside of the respective conveyor belt and may give the respective conveyor belt a desired shape. By taking this measure, it is for instance possible that the converging and departing of the two conveyor belts relative to one another at different sections may be achieved.

In an embodiment, the supply unit may comprise a first supply channel in communication with the first conveyor belt adapted for conveying the first tablet body (or a queue of first tablet bodies) via a first supply channel to the first conveyor belt. The supply unit may further comprise a second supply channel in communication with the second conveyor belt adapted for conveying the second tablet body (or a queue of second tablet bodies) via the second supply channel to the second conveyor belt. In one embodiment, the supply channels may be tubes enclosing a lumen along which the tablet bodies may be transported. According to another exemplary embodiment, the supply channels may be through-holes formed in a solid body such as a plate. By bringing the supply channels in communication with the respective conveyor belt, it is possible that, making use of the gravitational force exerted on the tablet bodies, the earliest tablet body in a queue is inserted from the respective supply channel into a corresponding accommodation space of the conveyor belt. Thus, the continuous rotation of the conveyor belts may be combined with a structurally simple provision of the supply channels.

Still referring to the previously described embodiment, the supply channels may be arranged for conveying the respective tablet body towards the respective conveyor belt driven by a gravitational force. Thus, the supply channels may be arranged along a path which has at least a vertical component so that the gravitational force acting on a tablet body provides for an automatic feeding of the tablet bodies queuing along the respective supply channel.

In an embodiment, the adhering unit may comprise an adhesive supply channel arranged for conveying the adhesive towards the adhering position driven by a gravitational force. Therefore, an adhesive material may also be transported at least partially along a direction of the gravitational force through the adhesive supply channel. The adhesive supply channel, together with one or both of the supply channels having the tablet bodies may form a bifurcated structure so that, at a position in which the adhesive supply channel is in fluid communication with the corresponding tablet body supply channel, adhesive material may be applied to the tablet body. This may be performed directly upstream of the converging section of the conveyor belts so that undesired adhesion between other portions of the apparatus may be safely prevented.

In an embodiment, the supply channels and the adhesive supply channels may be integrally formed within a support plate. For instance, it is possible to form through-holes in a support plate to form all supply channels of the apparatus.

Additionally or alternatively, it is possible that the first conveyor belt and the second conveyor belt are mounted to such a support plate. For example, mounting structures for mounting drive rolls and/or shaping rolls may be arranged on such a plate, whereas the conveyor belts may be guided along the rolls. This may contribute to the formation of a compact structure.

In an embodiment, the temperature adjustment unit may be arranged at the spatially converging portion of the first conveyor belt and the second conveyor belt. Therefore, precisely at the position where the tablet bodies are merged, the temperature adjustment is performed.

Still referring to the previously described embodiment, the temperature adjustment unit may comprise one or more cooling brackets having at least one cooling channel formed therein and being adapted so that a cooling agent (such as a cooling fluid) may be conducted through the at least one cooling channel for cooling the formed multi-layer tablet. For instance, it is possible that a respective cooling bracket is arranged within each of the conveyor belts close to the converging position. Thus, the cooling brackets may not only promote the compressing of the tablet bodies to one another due to the conversion in combination with the rotation of the conveyor belts, but additionally they may also locally apply cooling energy to the compressed tablet. Therefore, rapid cooling, for instance solidification of a liquid adhesive, may be achieved.

More specifically, the temperature adjustment unit may comprise a first cooling bracket within the first conveyor belt. The temperature adjusting unit may further comprise a second cooling bracket within the second conveyor belt. The first cooling bracket and the second cooling bracket may be arranged relative to one another to apply a compressing force on the first tablet body on the first conveyor belt and on the second tablet body on the second conveyor belt at the converging position. Therefore, the cooling brackets may not only serve for providing cooling energy, but they also contribute with their arrangement to the application of the pressure force.

Still referring to the previously described embodiment, the first cooling bracket and the second cooling bracket may be arranged to form a conically-shaped passageway for the first conveyor belt and the second conveyor belt. With such a conically-shaped passageway, the compression force may be maximized at a certain position so as to obtain a stable connection of the tablet bodies at the adhesive section.

The apparatus may comprise a tablet ejection unit adapted for ejecting a tablet from two corresponding accommodation spaces of the first conveyor belt and the second conveyor belt at a lower reverse position of the first conveyor belt and the second conveyor belt. Therefore, after having converged, the conveyor belts may reverse their motion direction from a downward motion to an upward motion and may at the same time allow ejection of the formed multi-layer tablets. The conveyor belts may then run back to an upper position where the engagement of new tablet bodies, supply of new adhesive and compression of the tablet bodies for forming a new multi-layer tablet is enabled.

In an embodiment, each of the first conveyor belt and the second conveyor belt has a vertical section and a connected slanted section (so as to form a V-shape), wherein the vertical sections are arranged parallel and juxtaposed (i.e. next) to one another so as to form a Y-shape together with the slanted sections. The terms "vertical" and "slanted" relate to a configuration of the apparatus during normal use. The vertical sections may be arranged at a small distance from one another ensuring that the paired tablet bodies accommodated in the vertical sections can be pressed onto one another when moving along the vertical sections. In contrast to this, along the slanted sections, the tablet bodies are moved with a significant distance from one another to successively approach one another. At an angled portion between the slanted section and the vertical section, the tablet bodies are then brought in contact to one another.

In an embodiment, the slanted sections are arranged to enclose an obtuse angle (i.e. an angle larger than 90°), particularly an angle in a range between about 120° and about 180°. Smaller angles are alternatively possible as well. However, if the angle is relatively large, the supply path is relatively long before the tablet bodies reach the vertical sections, thereby allowing that a number of further elements (such as an adhesive supply unit, a heating unit, a monitoring unit, etc.) can be arranged at the apparatus upstream of the vertical sections.

In an embodiment, the supply unit is adapted for providing the first tablet body at an uppermost position (i.e. at a highest vertical position) of the slanted section of the first conveyor belt and for providing the second tablet body at an uppermost position (i.e. at a highest vertical position) of the slanted section of the second conveyor belt. In this case, there is a spatially long path between the supply position of the tablet bodies to the respective conveyor belt and the adhering position, so that this path can be used for controlling the system, for instance by heating it or by applying the adhesive.

In an embodiment, the adhering unit is adapted for providing the adhesive at the slanted section of at least one of the first conveyor belt and the second conveyor belt. In one embodiment, only one of the tablet bodies to be connected can be applied with adhesive material. In another embodiment, an adhesive can be applied to both (or more than two) tablet bodies to be connected.

In an embodiment, the adhering unit has an adhesive heating unit adapted for heating the adhesive material prior to its supply to at least one of the tablet bodies in the first conveyor belt and/or the second conveyor belt. Such an embodiment may be advantageous, since the viscosity and the adhesion capabilities of the adhesive may be rendered more appropriate for a secure connection of the tablet bodies by heating the adhesive to an appropriate temperature. This is particularly advantageous when using an adhesive being solid at room temperature which is be heated and thereby brought to a liquid phase prior to the supply to a tablet body in a respective conveyor belt.

In an embodiment, the adhering unit has a micro-metering device. A micro-metering device may be denoted as a dosing unit providing an amount of adhesive with a metering accuracy of 1 µl or better. By such a micro-metering device, precise metering of the amount of adhesive to be applied can be ensured.

In an embodiment, the vertical section of at least one of the first conveyor belt and the second conveyor belt has a cooling zone adapted for cooling the multi-layer tablet in accordance with a predefined cooling pattern. Thus, after the connection of the tablet bodies in the vertical section, rapid cooling and therefore curing or solidifying of the adhesive may be performed by a corresponding cooling unit arranged along the vertical sections, before the multi-layer tablet is ejected.

In an embodiment, the vertical section of the first conveyor belt and/or the second conveyor belt has a pressure adjustment unit adapted for adjusting a pressure with which the first tablet body and the second tablet body are pressed together for forming the multi-layer tablet. Applying a sufficiently large or predefined pressure may additionally promote the adhesion connection. For instance, the distance between the vertical sections may be adjusted, since this distance has an impact on the pressure applied to a pair of tablet bodies (located in the conveyor belts in spatial alignment to one another) after being brought in connection to one another.

In an embodiment, the adhering unit comprises a screenprint device adapted for screenprinting the adhesive onto the first tablet body and/or the second tablet body. Screen printing is a printing technique that may use a woven mesh or the like to support an ink-blocking stencil. The attached stencil forms open areas of mesh that transfer printable adhesive which can be pressed through the mesh as a sharp-edged image onto one or more corresponding tablet bodies. A roller or the like may be moved across the screen stencil, forcing or pumping adhesive past the threads of the woven mesh in the open areas. Screenprinting has turned out to be a very effective way of applying adhesive material to a tablet body.

In an embodiment, the adhering unit comprises a roller device adapted for rolling (or calender coating) the adhesive on the first tablet body and/or the second tablet body. By rolling or calender coating the adhesive on the respective tablet body a specifically intense interaction between the adhesive material and the tablet material may be ensured. Calender coating can be denoted as a type of roller coating that is actually a laminating operation. The adhesive coating may be joined with the corresponding tablet body by one or more rollers.

In an embodiment, the adhering unit comprises an adhesive platelet application device adapted for heating and applying an adhesive platelet as the adhesive to the first tablet body and/or the second tablet body. Thus, even solid adhesive pellets or disks (solid at room temperature, for instance of wax material) may be used as adhesive. Only directly prior to the connection of the tablet bodies, the adhesive platelet may be heated to be brought into an adhering phase.

In the following, further exemplary embodiments of the method will be explained. However, these embodiments also apply to the apparatus and the tablet.

The method may comprise compressing the first powder comprising the first pharmacologically active substance to form the first tablet body. Furthermore, the method may comprise compressing the second powder comprising the second pharmacologically active substance to form the second tablet body. Hence, prior to the connection of the tablet bodies to one another, the tablet bodies may be formed individually by compression of powder.

In an embodiment, the adhesive may comprise wax, gelatine and/or starch. These materials have already been officially approved as substances usable for medicaments. It is possible that the adhesive (such as gelatine and/or starch) is dissolved in a solvent such as water. The present inventors have recognized that such a selection of the adhesive material may allow to prevent any negative influence on the cores of the tablet bodies carrying the physiologically active substances. With such materials, any dissolution and undesired mixture of the individual tablet bodies may be prevented. Furthermore, it is desirable that the adhesive material does not contain too large amounts of water which could be absorbed by the cores of the tablet bodies, so that a decomposition of the tablet bodies or the multi-layer tablet may be prevented. Also this requirement may be met by the mentioned materials for the adhesive.

Particularly the use of a wax, for instance bees wax, has turned out to be highly advantageous because it may be used for a solventless firm adhesion of the tablet bodies without the necessity to use a liquid solvent such as water, thereby preventing the tablet bodies from any disintegration caused by a liquid solvent. By heating solid wax (bees wax has a melting point of about 60 °C to 70 °C) prior to the adhesion and by subsequently cooling the wax after the adhesion, a fast and stable connection with the tablet bodies may be achieved. Furthermore, the evaporation of solvents (for instance water in a core of the tablet) can be prevented by a fast heating before the connection and a fast cooling thereafter. Hence, a wax material allows to rapidly dry the adhesion position to thereby increase the robustness of the multilayer tablet directly after ejection from the manufacturing apparatus.

The term "wax" may denote a substance secreted by bees and used in constructing their honeycombs. The term has come to refer more generally to a class of substances with properties similar to bees wax, i.e. which is plastic at normal ambient temperature of 20 °C, a melting point above 45 °C, is insoluble in water, is hydrophobic, and is classified as a lipid. Wax may have a relatively low viscosity when melted.

However, also other biocompatible adhesive materials can be used such as a fibrin adhesive or a chemical substitute thereof.

In an embodiment, the adhering may comprise heating the adhesive to liquefy the adhesive. The heating temperature depends on the used materials, particularly of the material of the adhesive. Subsequently, an interaction between the liquefied adhesive and the first tablet body and the second tablet body may be initiated. Hence, the adhesive applied for instance to one of the first tablet bodies may be contacted with the other tablet body. Subsequently, the adhesive may be cooled to solidify the adhesive. The precise temperature used for cooling depends on the material of the used components, particularly of the material of the adhesive.

Particularly, the promotion of drying of the adhesive after the adhering of the first tablet body and the second tablet body together by the adhesive may be advantageous.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.
Fig. 1 to Fig. 4 schematically show procedures performed during a method of manufacturing a tablet according to an exemplary embodiment of the invention.
Fig. 5 to Fig. 10 show an apparatus for manufacturing a tablet according to an exemplary embodiment of the invention in different operation states.
Fig. 11 to Fig. 14 show different components of an apparatus for manufacturing a tablet according to an exemplary embodiment of the invention.
Fig. 15 and Fig. 16 are different views of an apparatus for manufacturing a tablet according to still another exemplary embodiment of the invention.
Fig. 17 to Fig. 22 show cross-sectional views of different tablets according to exemplary embodiments of the invention.
Fig. 23 shows an adhesive metering unit of an apparatus for manufacturing a tablet according to an exemplary embodiment of the invention.
Fig. 24 to Fig. 27 are different views of apparatuses for manufacturing a tablet according to yet other exemplary embodiments of the invention.

The illustrations in the drawings are schematical. In different drawings, similar or identical elements are provided with the same reference signs.

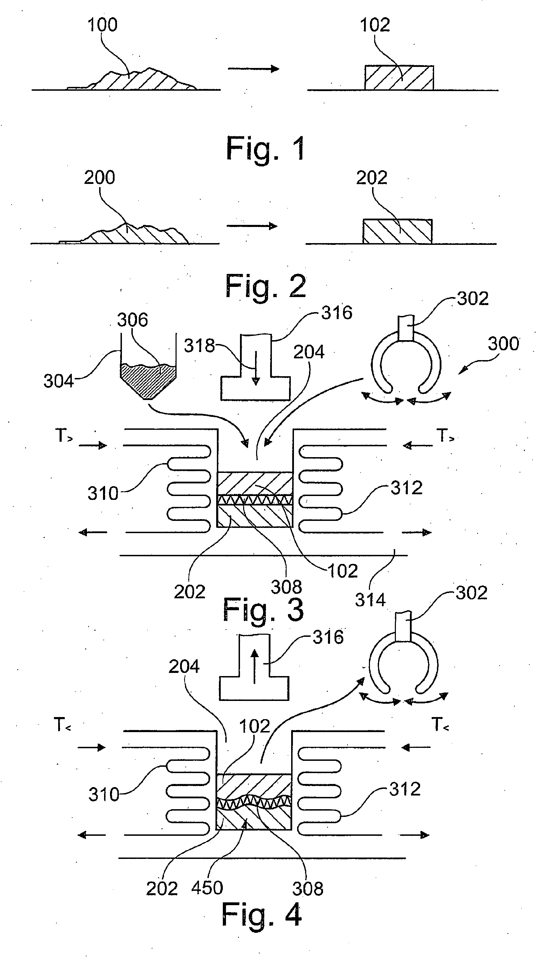

In the following, referring to Fig. 1 to Fig. 4, a method for manufacturing a tablet 450 shown in Fig. 4 according to an exemplary embodiment of the invention will be explained.

Referring to **Fig. 1**, a first powder 100 is shown which has a first pharmacologically active substance included therein. A first tablet body 102 is formed by compressing the first powder 100.

In a similar manner, as shown in **Fig. 2**, a second powder 200 is provided which comprises a second pharmacologically active substance. The pharmacologically active substances of the powders 100, 200 may differ. As shown schematically in Fig. 2, a procedure is applied to form a second tablet body 202 by compressing the second powder 200.

**Fig. 3** shows an arrangement 300 by which the tablet 450 according to an exemplary embodiment of the invention can be formed based on the first tablet body 102 and the second tablet body 202.

The arrangement 300 comprises a gripper 302 which is adapted for firstly gripping the second tablet body 202 and for inserting the second tablet body 202 into an accommodation space 204 of the arrangement 300. Subsequently, a capillary 304 accommodating a biocompatible adhesive material 306 therein is guided towards the accommodation space 204 to supply adhesive material 308 of a predefined volume onto a top surface of the second tablet body 202. Subsequently, the gripper unit 302 grips the first tablet body 102 and inserts it into the accommodation space 204 on top of the adhesive 308.

After having applied this adhesive 308 between the tablet bodies 102, 202, a heating fluid such as hot water at a temperature T_{>} may be guided through meandrically shaped heating channels 310, 312 of a support structure 314 of the arrangement 300. Thus, the viscosity of the adhesive 308 may be reduced for supporting adhesion between the tablet bodies 102, 202 via the adhesive 308. Subsequently, a piston 316 may be used and moved along a direction indicated by an arrow 318 to apply pressure to the adhesive 308 and the tablet bodies 102, 202 sandwiching the adhesive 308. Thus, an adhering connection between the tablet bodies 102, 202 may be provided by the adhesive 308.

As can be taken from **Fig. 4**, a cooling liquid at a temperature T_{<} < T_{>} may now be guided through the meandric fluid lines 310, 312 to again solidify the adhesive 308. After this, the gripper 302 may be used to remove the readily formed tablet 450 from the accommodation space 204.

In the following, referring to Fig. 5 to Fig. 10, an apparatus 500 for manufacturing a tablet according to an exemplary embodiment of the invention will be explained.

The apparatus 500 comprises a supply unit formed of multiple components which will be explained below in more detail and which is adapted for supplying a first tablet body 102 (as shown in Fig. 1) and a second tablet body 202 (as shown in Fig. 2). An adhering unit which will be described below in more detail is provided for forming a tablet based on the tablet bodies 102, 202 to be connected by an adhesive material as will be described below in more detail.

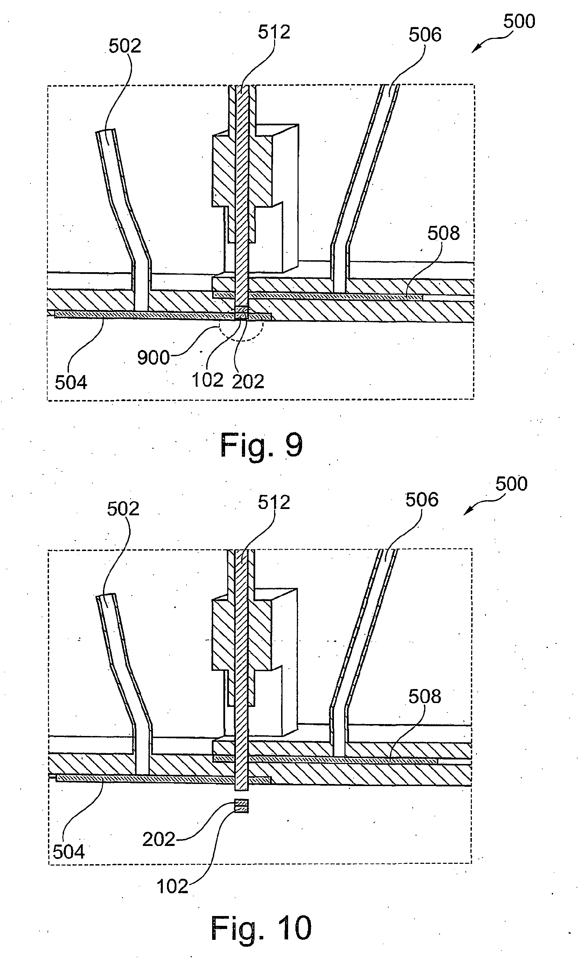

As can be taken from **Fig. 5**, the supply unit comprises a first supply channel 502 and a first feeder mechanism 504 adapted for conveying the first tablet body 102 towards an adhering position as indicated with reference numeral 900 in Fig. 9. Furthermore, the supply unit comprises a second supply channel 506 and a second feeder mechanism 508 adapted for conveying the second tablet body 202 towards the adhering position 900 at the adhering unit. The supply channels 502, 506 are arranged almost vertically so that a gravitational force indicated by the g-vector 510 promotes conveying the tablet bodies 102, 202 to the respective feeder mechanisms 504, 508.

As can further be taken from Fig. 5, the feeder mechanisms 504, 508 are both arranged for horizontally shifting the respective tablet body 102, 202 towards the adhering position 900 at the adhering unit.

Moreover, a press mechanism is provided which is adapted for applying a predefined pressure to the tablet bodies 102, 202 and the adhesive arranged between the tablet bodies 102, 202 at the adhering position 900. The press mechanism comprises a movable piston 512 adapted for applying the predefined pressure along a compressing direction which is perpendicularly arranged relative to the horizontal sliding direction of the feeder mechanisms 504, 508.

Fig. 5 shows a basic position of apparatus 500 in which the tablet bodies 102, 202 are supplied via the supply channels 502, 506 and are further transported to a destination by the respective feeder mechanisms 504, 508. It should further be mentioned that, in the embodiment of Fig. 5 to Fig. 10, a temperature adjustment unit as well as an adhesion supply metering unit are integrated within the movable piston 512. In other words, the movable piston 512 may be heated or cooled, and adhesive material may be supplied to the adhering position 900 via a channel formed within the movable piston 512.

As can be taken from **Fig. 6**, the first feeder mechanism 504 is operated to bring the first tablet body 102 towards the adhesion position 900.

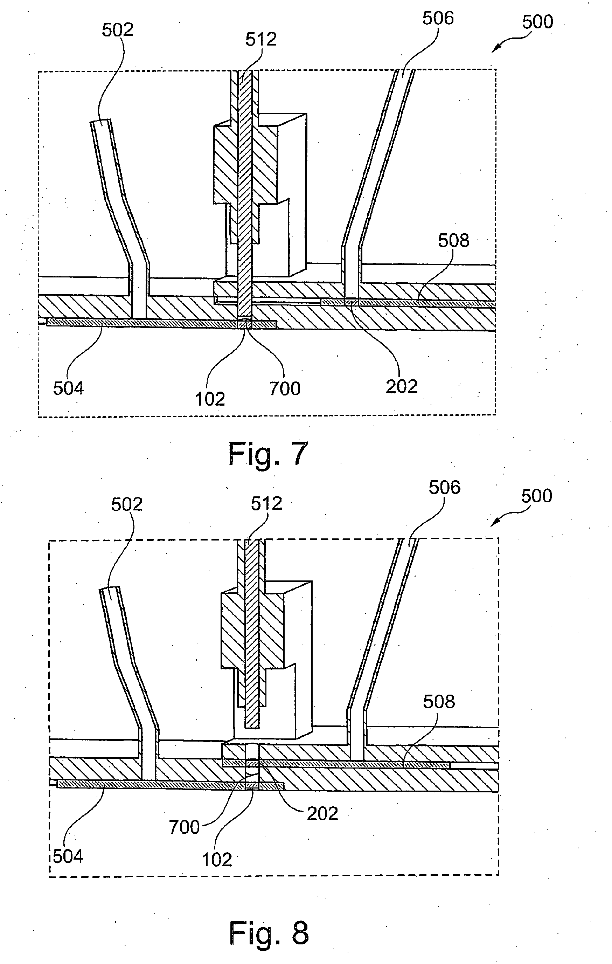

As shown schematically in **Fig. 7**, adhesive material 700 is supplied through the interior channel within the piston 512 with a metered quantity towards the first tablet body 102.

As can be taken from **Fig. 8**, the second feeder mechanism 508 is operated to bring also the second tablet body 202 towards the adhesion position 900.

As can be taken from **Fig. 9**, the movable piston 512 is lowered again so as to apply a predefined pressure to the tablet bodies 102, 202 having the adhesive material 700 in between.

As can be taken from **Fig. 10**, the manufactured tablet formed by the tablet bodies 102, 202 connected via the adhesive material 700 is ejected from the apparatus 500.

In the following, referring to Fig. 11 to Fig. 14, an apparatus 1100 for manufacturing a tablet according to another exemplary embodiment of the invention will be explained.

The apparatus 1100 comprises a rotatable wheel 1102 forming part of a turret mechanism. As can be taken from Fig. 11, the rotatable wheel 1102 is disk-shaped with a central bore 1104 and is configured to perform a rotation, as indicated by arrow 1105. Along a circumference of the wheel 1102, a plurality of equidistantly spaced, equally sized and equally designed accommodation spaces 1106 are formed as recesses in rotatable wheel 1102 and are to be rotated as well.

The supply unit comprises a first supply channel 1108 which is configured for supplying a first tablet body 102 towards one of the accommodation spaces 1106. Moreover, the supply unit comprises a second supply channel 1110 having an internal lumen through which the second tablet body 202 may be guided towards the same accommodation space 1106 in which also the first tablet body 102 is inserted. In order to allow the tablet bodies 102, 202, which can be provided by a reservoir (not shown in Fig. 11), to be approached to one another, the two supply channels 1108, 1110 are basically Y-shaped so that the channels 1108, 1110 are angled relative to one another at an inlet position and are in parallel to one another at an outlet position.

The supply channels 1108, 1110 act as a stator and remain stationary fixed. In contrast to this, the wheel 1102 is rotated by a not shown drive mechanism to thereby bring successively different ones of the accommodation spaces 1106, one after the other, in alignment with the tablet body outlet ends 1112 of the supply channels 1108, 1110. Thus, it is possible to fill in a plurality of tablet bodies into each of the supply channels 1108, 1110 which are successively inserted pairwise into the accommodation spaces 1106 for being adhered to one another, so that a mass production of tablets is possible. In other words, one accommodation space 1106 after the other will be brought in temporary alignment with the tablet body outlet 1112 to be filled with two bodies.

A tablet ejection position is located at an angular position of the wheel 1102 which opposes the portion of the wheel 1102 which is in alignment with the supply channels 1108, 1110. In other words, a tablet 450 formed by adhering and compressing the tablet bodies 102, 202 will be ejected after a rotation of about 180° within the wheel 1102. The tablet ejection unit is illustrated schematically with reference numeral 1114 in Fig. 11.

The filling of the turret mechanism is performed by the two separate supply channels 1108, 1110. During rotation of the wheel 1102, the previously heated tablets 450 can be cooled efficiently by convection. After a rotation of, in the shown embodiment, 180°, the already manufactured tablet 450 is ejected. Of course, other angular values are possible. The ejection angle may be in a range between 120° and 240°, more particularly in a range between 150° and 210°.

In an embodiment, the circumference of the wheel 1102 is guided with some clearance within a housing so that the tablets 450 are prevented from falling out of the accommodation spaces 1106 after the compressing performance during the rotation of the wheel 1102. The housing which may also include the tablet ejection unit 1114 has an opening at the lowermost position which allows the tablets 450 to fall out of the apparatus 1100 due to the gravitational force indicated by the g-vector in Fig. 11.

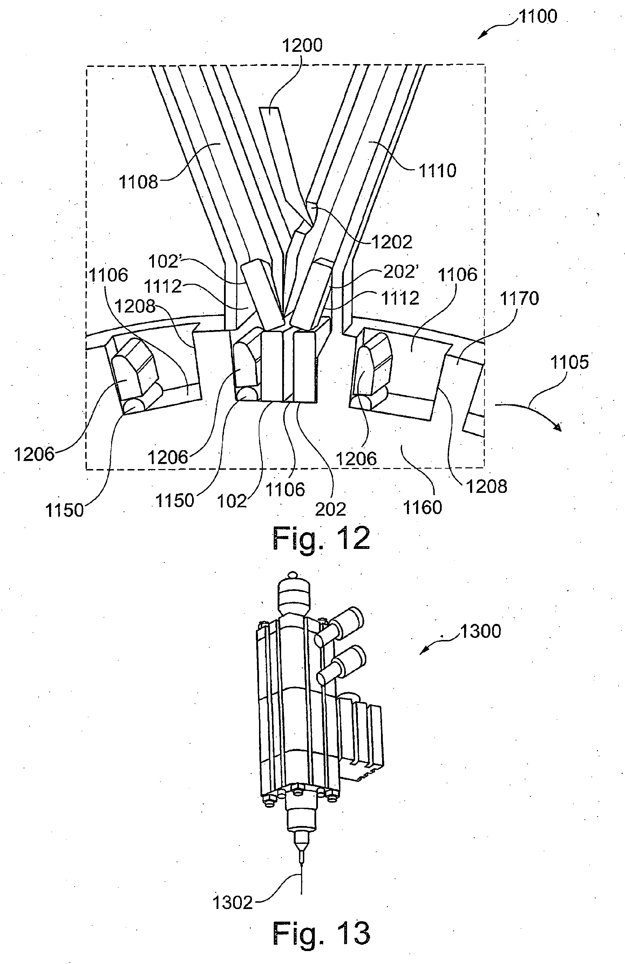

Now referring to **Fig. 12** and **Fig. 13**, the adhering unit of the apparatus 1100 is shown in more detail which comprises an adhesive metering valve 1300 which can impact on the apparatus 1100 at a position schematically illustrated with reference numeral 1200 in Fig. 12. The adhesive metering valve 1300 is arranged for supplying the adhesive material to the second tablet body 202 at a position in the second supply channel 1110 which is directly upstream of a boundary between the second supply channel 1110 and the respective one of the plurality of accommodation spaces 1106 being presently in alignment with the supply channels 1108, 1110. In other words, a bore may be formed in the second supply channel 1110, and the adhesive supply tip 1302 of the adhesive metering valve 1300 can be inserted via the recess 1202 into the second supply channel 1110 to provide a predefined amount of adhesive to the second tablet body 202. The adhesive is applied by the micrometering valve 1300 directly before the second tablet body 202' is inserted into the turret.

Many alternatives are possible to the arrangement shown in Fig. 12. For instance, the supply of adhesive may also be provided additionally or alternatively through a hole in the first supply channel 1108. It is also possible that the adhesive supply is integrated in the rotatable wheel 1102. In this scenario, the adhesive is applied when the tablet bodies 102, 202 are already positioned within one of the accommodation spaces 1106.

As can further be taken schematically from Fig. 12, many tablet bodies can be guided through each of the supply channels 1108, 1110 forming a queue of subsequent tablet bodies. For instance, in the example shown in Fig. 12, an earlier tablet body 102 has already been inserted into the aligned accommodation space 1106, whereas a later tablet body 102' still waits to be inserted in one of the accommodation spaces 1106. A similar scenario is shown for the second supply channel 1110 in which the tablet body 202 has already been inserted into the accommodation space 1106 at the central position in Fig. 12, whereas a subsequent tablet body 202' waits to be inserted into a next accommodation space 1106 after a predefined angular rotation along a direction 1105.

As can further be taken from Fig. 12, the press mechanism for compressing the tablet bodies 102, 202 with the adhesive material sandwiched in between is integrated in each accommodation space 1106. This compression mechanism is realized by a movable abutment unit 1206 and an opposingly arranged abutment surface 1208.

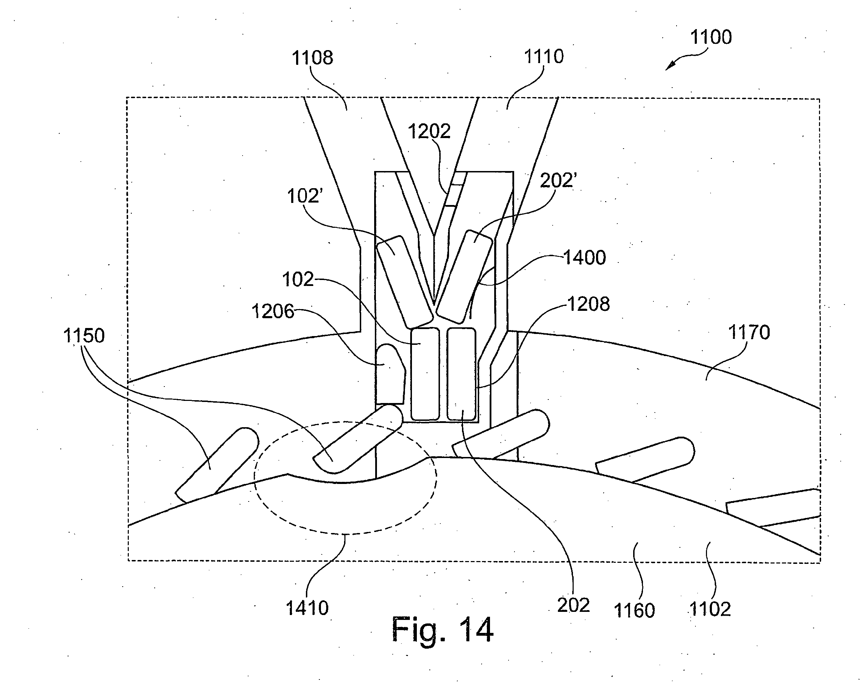

In an embodiment, the wheel 1102 of the turret mechanism can comprise two wheel sections, i.e. an inner spatially stationary section 1160 and a surrounding annular wheel section 1170 (comprising the accommodation spaces 1106) being rotatable. Upon rotation of the surrounding annular wheel section 1170 relative to the inner spatially stationary section 1160, a respective lever 1150 (which also rotates together with annular wheel section 1170) may trigger the respective movable abutment unit 1206 to initiate the pressing force exerted on the tablet bodies 102, 202 in a respective one of the accommodation spaces 1106 (compare also reference numeral 1410 in Fig. 14).

**Fig. 14** illustrates a retention leaf spring 1400 which is arranged at an outlet section of the second supply channel 1110. The retention leaf spring 1400 is arranged for exerting a retention force to tablet bodies, in the present scenario to a tablet body 202', at the later position inhibiting the tablet body 202' at the later position from advancing to the accommodation space 1106 before the tablet body 202 at the earlier position has left the position of alignment between the rotatable wheel 1102 and the supply channels 1108, 1110. The leaf spring 1400 is mounted at a side wall of the second supply channel 1110 and is biased. Due to the influence of the retention spring 1400, the tablet bodies 102, 202 are pressed only pairwise in the compressing chamber of the accommodation space 1106.

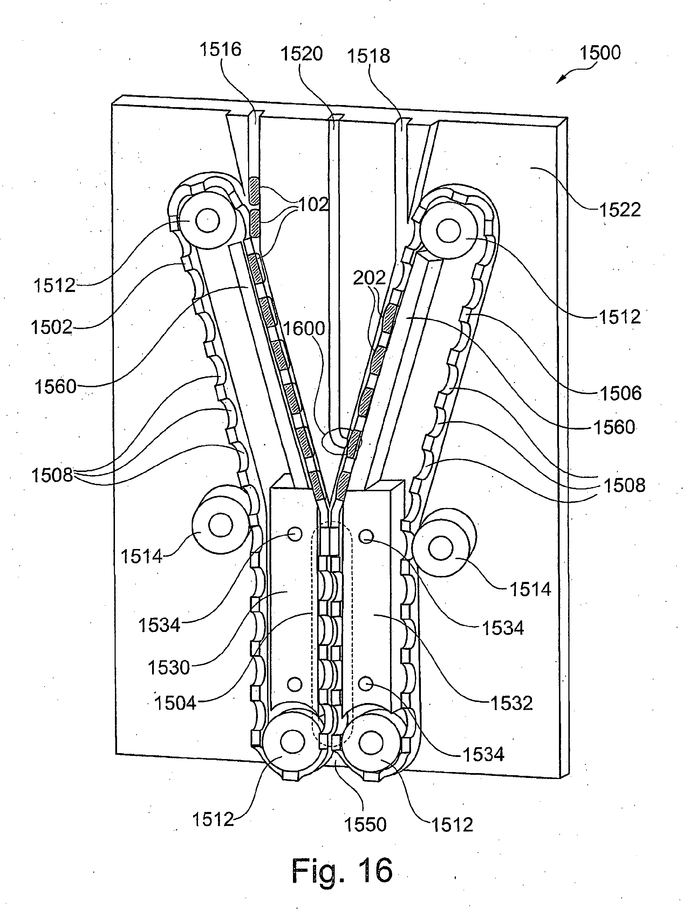

In the following, referring to Fig. 15 and Fig. 16, an apparatus 1500 for manufacturing a tablet according to still another exemplary embodiment will be explained.

As can be taken from **Fig. 15**, the apparatus 1500 comprises a first conveyor belt 1502 adapted for conveying a first tablet body to an adhering position 1504. A second conveyor belt 1506 is provided for conveying a second tablet body to the adhering position 1504. The first conveyor belt 1502 and the second conveyor belt 1506 are arranged to spatially converge at the adhering position 1504 so that the first tablet body and the second tablet body are pressed together by the converging first conveyor belt 1502 and second conveyor belt 1506 at the adhering position 1504. In other words, in an upper portion in Fig. 15, the distance between the conveyor belts 1502 and 1506 is larger than in a lower portion of Fig. 15.

Each of the conveyor belts 1502 comprises a plurality of accommodation spaces 1508 formed as recesses in the flexible belts 1502 and 1506. The accommodation spaces 1508 are arranged along a revolving motion trajectory of the respective conveyor belts 1502, 1506 indicated schematically by reference numeral 1510.

In the shown embodiment, two drive rolls 1512 are assigned to each of the conveyor belts 1502, 1506. The drive rolls 1512 are adapted for driving the respective conveyor belt 1502, 1506, wherein they can be rotated by a not shown drive mechanism such as a motor.

In addition, each of the conveyor belts 1502, 1506 has assigned a corresponding shaping roll 1514 which presses from an outside position towards the respective conveyor belt 1502, 1506 for defining a shape of the respective conveyor belt 1502, 1506.

Furthermore, the supply unit of the apparatus 1500 comprises a first supply channel 1516 and a second supply channel 1518. The first supply channel 1516 is in communication with the first conveyor belt 1502, as shown in more detail in **Fig. 16**. As can also be taken from Fig. 16, the second supply channel 1518 is in communication with the second conveyor belt 1506. Hence, a first tablet body may be transported via the first supply channel 1516 and an accommodation space 1508 of the first conveyor belt 1502 to the adhering position 1504. In a similar way, a second tablet body may be transported via the second supply channel 1518 and an accommodation space 1508 of the second conveyor belt 1506 to the adhering position 1504. The supply channels 1516, 1518 are almost vertically oriented so that a gravitational force, as indicated by the g-vector in Fig. 15, automatically forwards the tablet bodies towards the respective conveyor belt 1502, 1506, as can be seen in Fig. 16.

Moreover, the adhering unit of the apparatus 1500 comprises an adhesive supply channel 1520 which can be best seen in Fig. 16. Via the adhesive supply channel 1520, the adhesive can be transported towards the second conveyor belt 1506 in order to apply adhesive material to a surface of the second tablet bodies being transported along the second conveyor belt 1506. In the adhesion position 1504, the surface of the second tablet bodies having applied the adhesive material is brought in interaction with the first tablet body for promoting the adhesion.

As can be taken from Fig. 15 and Fig. 16, the supply channels 1516, 1518 as well as the adhesive supply channel 1520 are integrally formed within a support plate 1522. Furthermore, the first conveyor belt 1502 and the second conveyor belt 1506 are mounted to the support plate 1522 via the rolls 1512, 1514. This results in a compact design.

As can furthermore be taken from Fig. 15, a plurality, in the shown embodiment two, conveyor belts are arranged parallel to one another and behind one another. In other words, two conveyor belts having separate accommodation spaces 1508 are driven at the same time by the rolls 1512. This increases the throughput when producing tablets.

Fig. 15 furthermore shows a first cooling bracket 1530 and a second cooling bracket 1532 for cooling the finished tablets directly after production. Each of the cooling brackets 1530, 1532 has a cooling channel 1534, wherein Fig. 15 shows a cooling fluid inlet at an upper portion and a cooling fluid outlet at a lower portion of the respective cooling brackets 1530, 1532. A cooling fluid such as cool water or the like can be conducted through the cooling channels 1534 for cooling the formed multi-layer tablet direct after production.

In addition to their cooling function, the cooling brackets 1530, 1532 are arranged relative to one another to apply a compressing force on the first tablet body on the first conveyor belt 1502 and the second tablet body on the second conveyor belt 1506. For this reason, the cooling brackets 1530, 1532 can be aligned relative to one another to form a conically shaped passageway for the first conveyor belt 1502 and the second conveyor belt 1506 in the adhering position 1504.

At a position 1550, the manufactured tablets fall out of the apparatus 1500 so that this position serves as a tablet ejection unit. The rolls 1512, 1514 act as belt bearings. Furthermore, belt slider plates 1560 are arranged for supporting the conveyor belts 1502, 1506. Within the supply channels 1516, 1518, the tablet bodies 102, 202 are pressed by their own weight into the hold conveyor belts 1502, 1506. The adhesive is applied on to the second tablet bodies at a position 1600 as shown in Fig. 16.

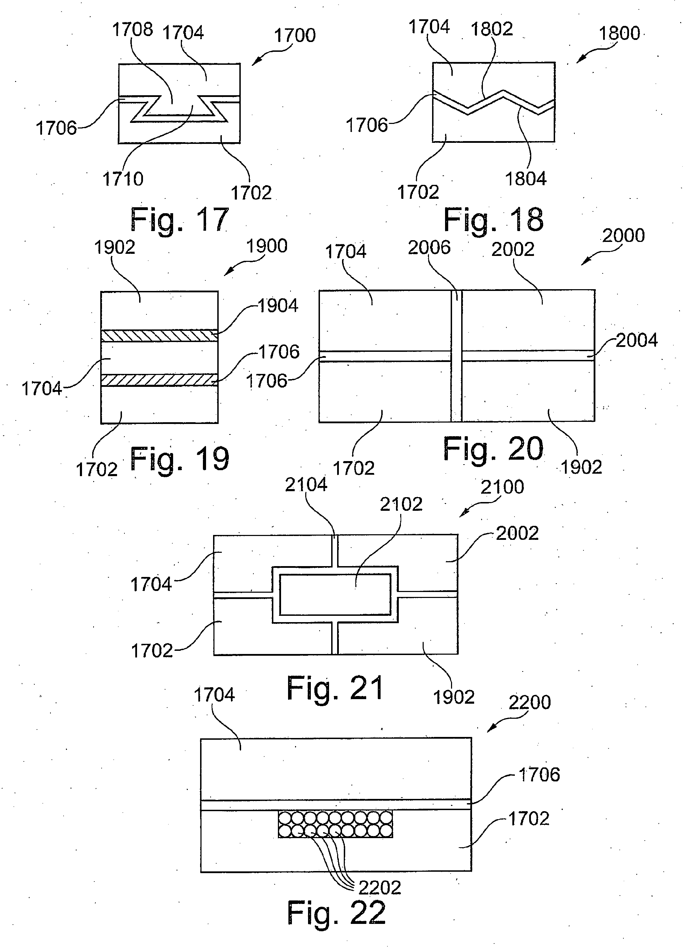

**Fig. 17** shows a cross-sectional view of a tablet 1700 according to an exemplary embodiment of the invention. Tablet 1700 is formed of two tablet bodies 1702, 1704 being connected to one another by an adhesive layer 1706. Tablet body 1702 is provided with a dovetail recess 1710, whereas tablet body 1704 is provided with a correspondingly shaped dovetail protrusion 1708. Dovetail recess 1710 and dovetail protrusion 1708 form cooperating engagement structures which are plugged together so that the tablet bodies 1702, 1704 are preconnected by a form closure providing support during subsequent adhering.

**Fig. 18** shows a cross-sectional view of a tablet 1800 according to another exemplary embodiment of the invention. Tablet 1800 is formed of two tablet bodies 1702, 1704 being connected to one another by an adhesive layer 1706 contacting two opposing, cooperating saw-tooth shaped surfaces 1802, 1804 of the tablet bodies 1702, 1704. Cooperating engagement structures, such as the saw-tooth shaped surfaces 1802, 1804, of the tablet bodies 1702, 1704 may promote an initial adhesion since a lateral sliding of the tablet bodies 1702, 1704 may be prevented.

**Fig. 19** shows a cross-sectional view of a tablet 1900 according to another exemplary embodiment of the invention. Tablet 1900 is formed of three tablet bodies 1702, 1704, 1902, adjacent ones of which being connected to one another by a respective adhesive layer 1706, 1904 contacting two opposing planar surfaces of the respective tablet bodies 1702, 1704, 1902. In the shown embodiment, the powder material of the tablet bodies 1702, 1704, 1902 may be different. For a reliable connection of the tablet bodies 1702, 1704, 1902, two different adhesive materials may be used for the adhesive layer 1706 and for the adhesive layer 1904.

**Fig. 20** shows a cross-sectional view of a tablet 2000 according to another exemplary embodiment of the invention. Tablet 2000 is formed of four tablet bodies 1702, 1704, 1902, 2002 being connected to one another by adhesive layers 1706, 2004, 2006. Tablet bodies 1702, 1704 are connected to one another by adhesive layer 1706. Tablet bodies 1902, 2002 are connected to one another by adhesive layer 2004. Then, the pairwise connected double tablet bodies 1702, 1704 and 1902, 2002 are connected to one another by adhesive layer 2006. The various adhesive layers form, in the cross-sectional view, a cross structure.

**Fig. 21** shows a cross-sectional view of a tablet 2100 according to another exemplary embodiment of the invention. Tablet 2100 is formed of five tablet bodies 1702, 1704, 1902, 2002, 2102 being connected to one another by an adhesive structure 2104. Adhesive material is applied to the adhesion surfaces of the various tablet bodies 1702, 1704, 1902, 2002, 2102. Then, the tablet bodies 1702, 1704, 1902, 2002, 2102 with adjusted surface topologies are put together and are connected by the adhesive structure 2104 to form the tablet 2100.

**Fig. 22** shows a cross-sectional view of a tablet 2200 according to an exemplary embodiment of the invention. Tablet 2200 is formed of two tablet bodies 1702, 1704 being connected to one another by an adhesive layer 1706. However, an accommodation recess is formed in tablet body 1702. The recess is filled with a plurality of pellets 2202 containing a pharmaceutically active agent. The pellets 2202 may be filled in the recess of the tablet body 1702, the adhesive material may be applied to the tablet body 1704, and the tablet body 1704 may then be attached to the filled tablet body 1702.

Fig. 17 to Fig. 22 show cross sections of various tablets. The individual aspects of Fig. 17 to Fig. 22 can of course be combined to form further tablet structures. Embodiments of the invention cover all different kinds of tablets and may have any desired shape comprising, but not limited to, a circular plan view, an oval plan view, a polygonal plan view, having rounded or sharp edges. Tablets according to embodiments of the invention can be made in virtually any shape, for instance round, oval or capsule shaped.

The individual tablet bodies may be provided with cooperating engagement structures so that the tablet bodies may be plugged together to achieve a form closure prior to adhering. For instance, the tablet bodies may be connected to one another by dovetailing.

In the following, exemplary materials regarding adhesives for manufacturing tablets by adhering the tablet bodies by the adhesive will be described. These materials were successfully tested in experiments.
Adhesive: Starch, pure, from rice:
   - Hersteller: Roth
   - Art. No.: 9368.1
   - Charge: 44989627
Adhesive: Gelatine, pure, Platin 240 Bloom
   - Producer: Roth
   - Art. No.: 4582.1
   - Charge: 030109572
Adhesive: Gelatine, pure, Gold 180 Bloom
   - Producer: Roth
   - Art. Nr.: 4274.1
   - Charge: 319108552
Adhesive: Dextrin, yellow, pure
   - Producer: Roth
   - Art. No.: 6777.1
   - Charge: 299108032
Adhesive: Bees wax, yellow:
   - Producer: Roth
   - Art. No.: 5830.2
   - Charge: 459103072
   - Dropping point: 61°C to 65°C

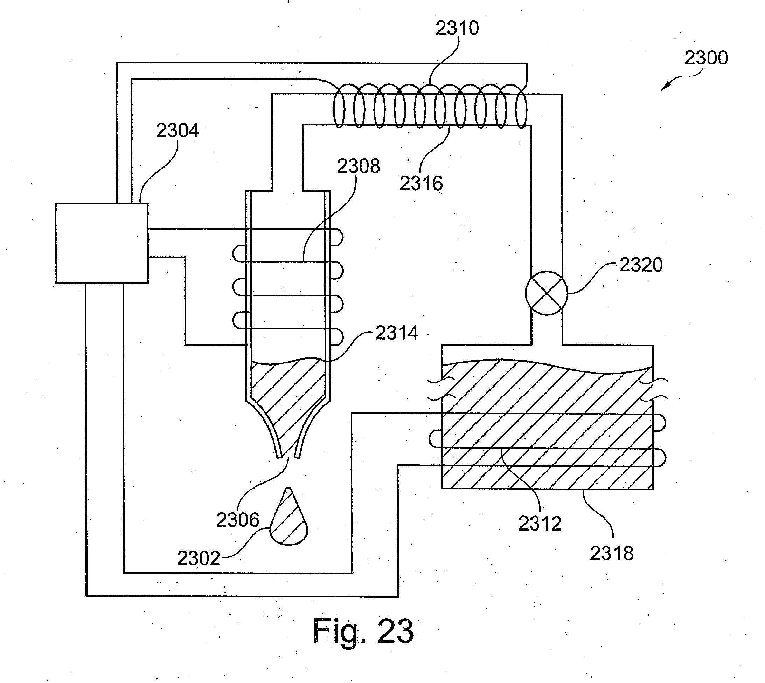

**Fig. 23** shows an adhesive metering unit 2300 of an apparatus for manufacturing a tablet (such as anyone shown in Fig. 5 to Fig. 16) according to an exemplary embodiment of the invention.

The adhesive metering unit 2300 is adapted for providing the adhesive with a predefined application rate, for instance in the form of a droplet 2302 of predefined volume or predefined mass, to at least one of the tablet bodies (not shown). As shown in Fig. 23, the adhesive metering unit 2300 comprises a temperature adjustment unit 2304 adapted to apply heat to the adhesive before ejection of the adhesive through orifice 2306. Therefore, it is possible to precisely control the viscosity of the adhesive before ejection, thereby also allowing to properly adjust the fluid flow properties before (and after) ejection. Ohmic heating coils 2308, 2310, 2312 are spirally wound around a capillary 2314 of the adhesive metering unit 2300, around a fluidic conduit 2316 between the capillary 2314 and an adhesive container 2318, and around the adhesive container 2318, respectively. A controllable valve 2320 is arranged between the capillary 2314 and the adhesive container 2318.

Fig. 23 does not show the mechanism of dispensing adhesive. However, such a dosing mechanism may be based on a piezoelement (not shown) acting on the capillary 2314. In this context, a mechanism may be implemented as the one disclosed in WO 2010/12470 A1.

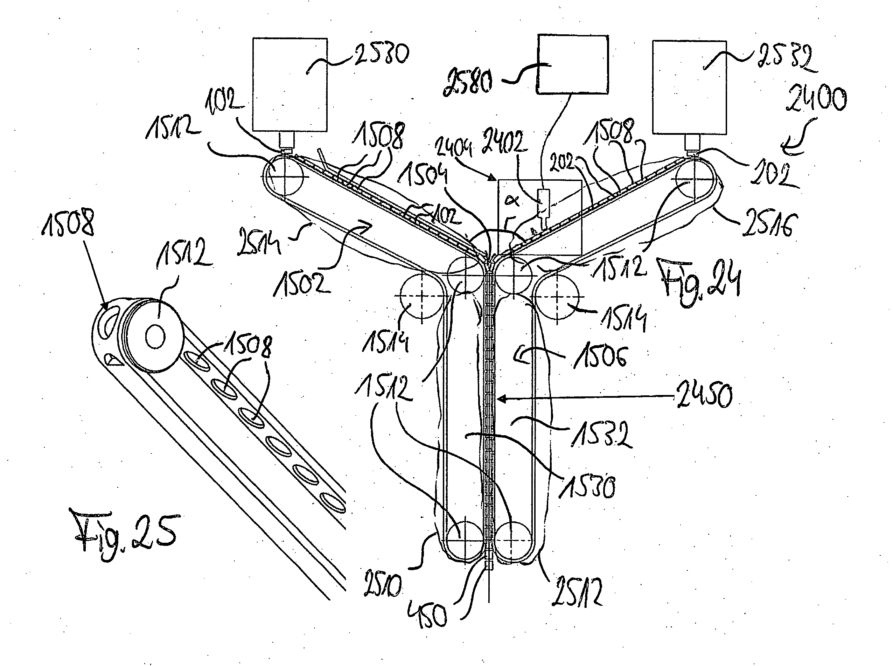

**Fig. 24** illustrates an apparatus 2400 according to another exemplary embodiment of the invention which is a modification of the embodiment shown in Fig. 15 and Fig. 16. **Fig. 25** shows an enlarged three-dimensional view of a portion of the apparatus 2400.

A difference between the apparatus 2400 on the one hand and apparatus 1500 on the other hand is that the two arms forming the conveyor belts 1502, 1506 are more elongate and further spaced apart from one another in Fig. 24 making it possible to apply the adhesive in other ways as shown in Fig. 15 and Fig. 16.

In the embodiment of Fig. 24, a metering unit 2402 is foreseen for a metered supply of adhesive material to tablet body 202 only. A control unit 2580 for controlling supply of adhesive material to the metering unit 2402 is foreseen as well. Furthermore, a heating unit 2404 is schematically shown and is configured for heating the adhesive material ejected by the metering unit 2402 prior to the application onto the respective tablet body 202. The heating unit 2404 may be integrated in the metering unit 2402 or may be a separate device. Hence, the embodiment of Fig. 24 and Fig. 25 has a microdosing unit 2402 for applying a metered amount of the adhesive material and has an optional heating unit 2404 for heating the applied adhesive (for instance wax).

After having adhered the tablet bodies 102, 202 to one another to thereby form multi-layer tablet 450, the multi-layer tablet 450 is guided along a predefined cooling line (see cooling brackets 1530, 1532) in vertical portions 2510, 2512 of the conveyor belts 1502, 1506. Parallel to the cooling procedure, the vertical portions 2510, 2512 of the conveyor belts 1502, 1506 act as a mechanism for adjusting the pressure for securely connecting the tablet bodies 102, 202. This pressure adjusting mechanism is operable by modifying the distance or gap between the vertical portions 2510, 2512.

Each of the two conveyor belts 1502, 1506 has a respective one of the vertical sections 2510, 2512 and has a corresponding one of slanted sections 2514, 2516. The vertical sections 2510, 2512 are arranged parallel to and spaced from one another. The slanted sections 2514, 2516 enclose an obtuse angle α of for instance 150°. Hence, as can be taken from Fig. 24, the conveyor belts 1502, 1506 together constitute an Y-shaped structure.

As can be taken from Fig. 24 as well, the supply of the first tablet body 102 to an accommodation space 1508 of the first conveyor belt 1502 and the supply of the second tablet body 202 to an accommodation space 1508 of the second conveyor belt 1506 is performed at an uppermost position of the respective slanted section 2514, 2516, i.e. at a reverse point at which the belt motion reverses from an upward motion to a downward motion. For the purpose of delivering the respective tablet body 102, 202, a first tablet supply unit 2530 and a second tablet body supply unit 2532 are provided. Tablet supply units 2530, 2532 can optionally also include a positioning unit, a sorting unit and/or a loading unit for the respective tablet bodies 102, 202 to be supplied to the respective conveyor belt 1502, 1506.

The accommodation recesses 1508 for receiving the tablet bodies 102 or 202 can be embodied as perforations of the conveyor belts 1502, 1506, or may alternatively be embodied as pockets, recesses, bores or presscuts in the respective conveyor belt 1502, 1506.

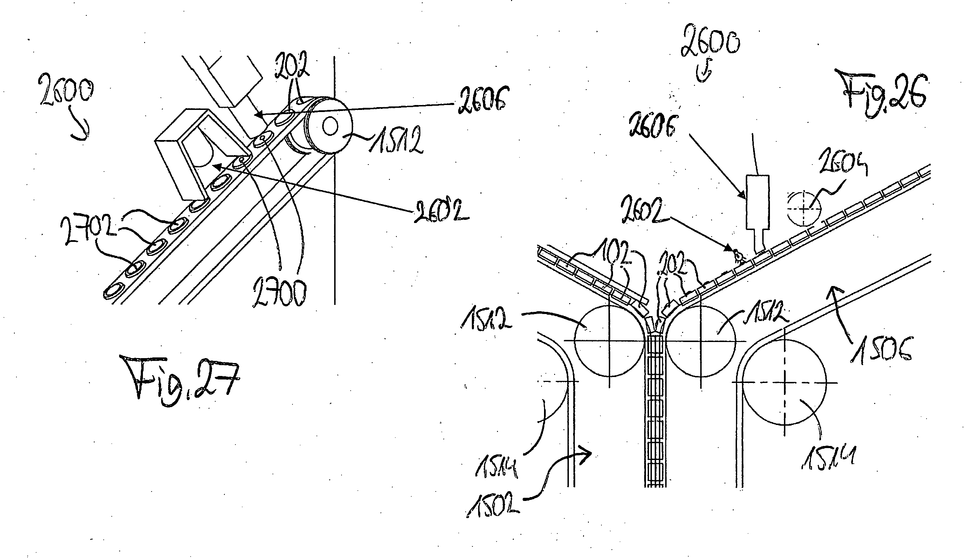

**Fig. 26** shows a first detailed view and **Fig. 27** shows a further detailed view of an apparatus 2600 according to another exemplary embodiment of the invention.

As can be taken from Fig. 26, the apparatus 2600 can comprise a heating unit 2602 configured for heating the applied adhesive material by means of irradiation, hot air or the like. Fig. 26 furthermore shows that, as an alternative to the microdosing unit 2402 described above referring to Fig. 24, it is also possible to apply the adhesive by another mechanism. For instance, a pharmaceutically admissible adhesive such as a solid adhesive platelet from wax can be applied by a platelet supply unit 2606 and can be melted by the supply of thermal heat. As can furthermore be taken from **Fig. 27**, the platelet supply unit 2606 can apply the adhesive in the form of a wax platelet 2700 to a tablet body 2700 which can then be heated by heating unit 2602 and can therefore be rendered soft or liquid so as to adhere to the tablet body 202 for connection of this tablet body 202 and subsequently to another tablet body 102 (not shown in Fig. 27).

A further alternative way of applying adhesive to a tablet body is by means of a rotating roller 2604, as shown schematically in Fig. 26. In the embodiment in which the roller 2604 is used, a film of adhesive material can be applied by the roller 2604, and optionally the heating element 2602 can be used for heating the applied adhesive (for instance wax).

It should be noted that the term "comprising" does not exclude other elements or steps.

Implementation of the invention is not limited to the preferred embodiments shown in the figures and described above. Instead, a multiplicity of variants are possible which use the solutions shown and the principle according to the invention.

## Claims

1. An apparatus (1500) for manufacturing a multi-layer tablet (450), the apparatus comprising:
a supply unit adapted for providing a first tablet body (102) comprising a compressed first powder (100) having a first pharmacologically active substance, and adapted for providing a second tablet body (202) comprising a compressed second powder (200) having a second pharmacologically active substance; and
an adhering unit for forming the multi-layer tablet (450) by adhering the first tablet body (102) and the second tablet body (202) together by an adhesive (308, 700); **characterized in that**
the supply unit comprises a first conveyor belt (1502) adapted for conveying the first tablet body (102) to an adhering position (1504) and comprises a second conveyor belt (1506) adapted for conveying the second tablet body (202) to the adhering position (1504), wherein the first conveyor belt (1502) and the second conveyor belt (1506) are arranged to spatially converge at the adhering position (1504) so that the first tablet body (102) and the second tablet body (202) are pressed together by the converging first conveyor belt (1502) and second conveyor belt (1506) at the adhering position (1504).

2. The apparatus (1500) of claim 1, wherein at least one of the first conveyor belt (1502) and the second conveyor belt (1506) comprises a plurality of accommodation spaces (1508) arranged along a motion trajectory of the respective conveyor belt and each being adapted for accommodating a respective tablet body.

3. The apparatus (1500) of claim 1 or 2, wherein at least one of the first conveyor belt and the second conveyor belt (1506) comprises at least one drive roll (1512, 1514) adapted for driving the respective conveyor belt.

4. The apparatus (1500) of any one of claims 1 to 3, wherein at least one of the first conveyor belt (1502) and the second conveyor belt (1506) comprises at least one shaping roll (1514) adapted for defining a shape of the respective conveyor belt.

5. The apparatus (1500) of any one of claims 1 to 4, wherein the supply unit comprises a first supply channel (1516) in communication with the first conveyor belt and adapted for conveying the first tablet body (102) via the first supple channel (1516) to the first conveyor belt (1502), and comprises a second supply channel (1518) in communication with the second conveyor belt (1506) and adapted for conveying the second tablet body (202) via the second supply channel (1518) to the second conveyor belt (1506).

6. The apparatus (1500) of claim 5, wherein the supply channels are arranged for conveying the respective tablet body towards the respective conveyor belt driven by a gravitational force.

7. The apparatus (1500) of any one of claims 1 to 6, wherein the adhering unit comprises an adhesive supply channel (1520) arranged for conveying the adhesive (308) towards at least one of the first tablet body (102) and the second tablet body (202) driven by a gravitational force.

8. The apparatus (1500) of claim 5 to 7, comprising a support plate (1522), wherein at least a part of the group consisting of the first supply channel (1516), the second supply channel (1518), and the adhesive supply channel (1520) is integrally formed within the support plate (1522), and/or wherein at least a part of the group consisting of the first conveyor belt (1502) and the second conveyor belt (1506) is mounted to the support plate (1522).

9. The apparatus (1500) of any one of claims 1 to 8, comprising a temperature adjustment unit (2304) adapted for adjusting, particularly at the adhering unit, a temperature of at least one of the group consisting of the first tablet body (102), the second tablet body (202), and the adhesive (308).

10. The apparatus (1500) of claim 9, wherein the temperature adjustment unit (2304) is adapted for heating at least one of the group consisting of the first tablet body (102), the second tablet body (202), and the adhesive (308) prior and/or during the adhering.

11. The apparatus (1500) of any one of claims 9 to 10, wherein the temperature adjustment unit (2304) is arranged at a spatially converging portion of the first conveyor belt (1502) and the second conveyor belt (1506).

12. The apparatus (1500) of claim 9 to 11, wherein the temperature adjustment unit (2304) comprises at least one cooling bracket having at least one cooling channel and being adapted so that a cooling agent is conductable through the at least one cooling channel.

13. The apparatus (1500) of any one of claims 1 to 12, wherein each of the first conveyor belt (1502) and the second conveyor belt (1506) has a vertical section (1510, 2512) and an angularly connected slanted section (2514, 2516), wherein the vertical sections (1510, 2512) are arranged parallel and juxtaposed to one another so as to form a Y-shape together with the slanted sections (2514, 2516).

14. The apparatus (1500) of claim 13, wherein the vertical section (1510, 2512) of at least one of the first conveyor belt (1502) and the second conveyor belt (1506) has a pressure adjustment mechanism adapted for adjusting a pressure according to which the first tablet body (102) and the second tablet body (202) are pressed together for forming the multi-layer tablet (450).

15. A method of manufacturing a multi-layer tablet (450) using an apparatus (1500) of any one of claims 1 to 14, the method comprising:
providing a first tablet body (102) comprising a compressed first powder (100) having a first pharmacologically active substance;
providing a second tablet body (202) comprising a compressed second powder (200) having a second pharmacologically active substance;
forming the multi-layer tablet (450) by adhering the first tablet body (102) and the second tablet body together by an adhesive (308, 700).

## Patentansprüche

1. Eine Vorrichtung (1500) zum Herstellen einer mehrlagigen Tablette (450), wobei die Vorrichtung aufweist:
eine Zufuhreinheit,
welche zum Bereitstellen eines ersten Tablettenkörpers (102) eingerichtet ist, welcher ein verdichtetes erstes Pulver (100) aufweist, welches eine erste pharmakologisch aktive Substanz hat, und
welche zum Bereitstellen eines zweiten Tablettenkörpers (202) eingerichtet ist, welcher ein verdichtetes zweites Pulver (200) aufweist, welches eine zweite pharmakologisch aktive Substanz hat; und
eine Klebeeinheit zum Bilden der mehrlagigen Tablette (450) mittels Zusammenklebens des ersten Tablettenkörpers (102) und des zweiten Tablettenkörpers (202) mittels eines Klebers (308, 700); **dadurch gekennzeichnet, dass**
die Zufuhreinheit ein erstes Förderband (1502) aufweist, welches zum Fördern des ersten Tablettenkörpers (102) zu einer Klebeposition (1504) eingerichtet ist, und ein zweites Förderband (1506) aufweist, welches zum Fördern des zweiten Tablettenkörpers (202) zu der Klebeposition (1504) eingerichtet ist,
wobei das erste Förderband (1502) und das zweite Förderband (1506) eingerichtet sind, um an der Klebeposition (1504) räumlich zu konvergieren, so dass der erste Tablettenkörper (102) und der zweite Tablettenkörper (202) mittels des konvergierenden ersten Förderbands (1502) und zweiten Förderbands (1506) an der Klebeposition (1504) zusammen gedrückt werden.

2. Die Vorrichtung (1500) gemäß Anspruch 1, wobei zumindest eines des ersten Förderbands (1502) und des zweiten Förderbands (1506) eine Mehrzahl von Aufnahmeräumen (1508) aufweist,
welche entlang einer Bewegungstrajektorie des entsprechenden Förderbands angeordnet sind, und
welche jeweils zum Aufnehmen eines entsprechenden Tablettenkörpers eingerichtet ist.

3. Die Vorrichtung (1500) gemäß Anspruch 1 oder 2, wobei zumindest eines des ersten Förderbands und des zweiten Förderbands (1506) zumindest eine Antriebsrolle (1512, 1514) aufweist, welche zum Antreiben des entsprechenden Förderbands eingerichtet ist.

4. Die Vorrichtung (1500) gemäß einem beliebigen der Ansprüche 1 bis 3 wobei zumindest eines des ersten Förderbands (1502) und des zweiten Förderbands (1506) zumindest eine Gestaltgebungsrolle (1514) aufweist, welche zum Definieren einer Gestalt des entsprechenden Förderbands eingerichtet ist.

5. Die Vorrichtung (1500) gemäß einem beliebigen der Ansprüche 1 bis 4, wobei die Zufuhreinheit
einen ersten Zufuhrkanal (1516) aufweist
in Kommunikation mit dem ersten Förderband und
eingerichtet zum Fördern des ersten Tablettenkörpers (102) mittels des ersten Zufuhrkanals (1516) an das erste Förderband (1502), und
einen zweiten Zufuhrkanal (1518) aufweist
in Kommunikation mit dem zweiten Förderband (1506) und eingerichtet zum Fördern des zweiten Tablettenkörpers (202) mittels des zweiten Zufuhrkanals (1518) an das zweite Förderband (1506).

6. Die Vorrichtung (1500) gemäß Anspruch 5, wobei die Zufuhrkanäle eingerichtet sind zum Fördern des entsprechenden Tablettenkörpers zu dem entsprechenden Förderband hin, angetrieben mittels einer Gravitationskraft.

7. Die Vorrichtung (1500) gemäß einem beliebigen der Ansprüche 1 bis 6, wobei die Klebeeinheit einen Kleberzufuhrkanal (1520) aufweist, welcher zum Fördern des Klebers (308) zu zumindest einem des ersten Tablettenkörpers (102) und des zweiten Tablettenkörpers (202) hin eingerichtet ist, angetrieben mittels einer Gravitationskraft.

8. Die Vorrichtung (1500) gemäß Anspruch 5 bis 7, aufweisend eine Stützplatte (1522),
wobei zumindest ein Teil der Gruppe, welche aus dem ersten Zufuhrkanal (1516), dem zweiten Zufuhrkanal (1518) und dem Kleberzufuhrkanal (1520) besteht, integral mit der Stützplatte (1522) gebildet ist, und/oder
wobei zumindest ein Teil der Gruppe, welche aus dem ersten Förderband (1502) und dem zweiten Förderband (1506) besteht, an der Stützplatte (1522) montiert ist.

9. Die Vorrichtung (1500) gemäß einem beliebigen der Ansprüche 1 bis 8, aufweisend eine Temperaturanpassungseinheit (2304), welche zum Anpassen, insbesondere an der Klebeeinheit, einer Temperatur von zumindest einer der Gruppe, welche aus dem ersten Tablettenkörper (102), dem zweiten Tablettenkörper (202) und dem Kleber (308) besteht, eingerichtet ist.

10. Die Vorrichtung (1500) gemäß Anspruch 9, wobei die Temperaturanpassungseinheit (2304) eingerichtet ist zum Heizen von zumindest einem aus der Gruppe, welche aus dem ersten Tablettenkörper (102), dem zweiten Tablettenkörper (202) und dem Kleber (308) besteht, vor und/oder während des Klebens.

11. Die Vorrichtung (1500) gemäß einem beliebigen der Ansprüche 9 bis 10, wobei die Temperaturanpassungseinheit (2304) an einem räumlich konvergierenden Abschnitt des ersten Förderbands (150) und des zweiten Förderbands (1506) eingerichtet ist.

12. Die Vorrichtung (1500) gemäß Anspruch 9 bis 11, wobei die Temperaturanpassungseinheit (2304) zumindest einen Kühlträger aufweist, welcher zumindest einen Kühlkanal hat und welche eingerichtet ist, so dass ein Kühlmittel durch den zumindest einen Kühlkanal durchführbar ist.

13. Die Vorrichtung (1500) gemäß einem beliebigen der Ansprüche 1 bis 12, wobei jeweils das erste Förderband (1502) und das zweite Förderband (1506) einen vertikalen Abschnitt (1510, 2512) und einen winkelig verbundenen abgeschrägten Abschnitt (2514, 2516) hat,
wobei die vertikalen Abschnitte (1510, 2512) parallel und nebeneinanderstehend zueinander angeordnet sind, um zusammen mit den abgeschrägten Abschnitten (2514, 2516) eine Y-Form zu bilden.

14. Die Vorrichtung (1500) gemäß Anspruch 13, wobei der vertikale Abschnitt (1510, 2512) zumindest eines des ersten Förderbands (1502) und des zweiten Förderbands (1506) einen Druckanpassungsmechanismus hat, welcher zum Anpassen eines Drucks eingerichtet ist, gemäß dem der erste Tablettenkörper (102) und der zweite Tablettenkörper (202) zusammengedrückt werden, um die mehrschichtige Tablette (450) zu bilden.

15. Ein Verfahren zum Herstellen einer mehrschichtigen Tablette (450), welches eine Vorrichtung (1500) gemäß einem beliebigen der Ansprüche 1 bis 14 verwendet, wobei das Verfahren aufweist:
Bereitstellen eines ersten Tablettenkörpers (102), welcher ein verdichtetes erstes Pulver (100) aufweist, welches eine erste pharmazeutisch aktive Substanz hat;
Bereitstellen eines zweiten Tablettenkörpers (202), welcher ein verdichtetes zweites Pulver (202) aufweist, welches eine zweite pharmazeutisch aktive Substanz hat;
Bilden der mehrschichtigen Tablette (450) mittels Zusammenklebens des ersten Tablettenkörpers (102) und des zweiten Tablettenkörpers (202) mittels eines Klebers (308, 700).

## Revendications

1. Appareil (1500) pour fabriquer un comprimé multicouche (450), l'appareil comprenant :
une unité d'alimentation conçue pour fournir un premier corps de comprimé (102) comprenant une première poudre comprimée (100) ayant une première substance pharmacologiquement active, et conçue pour fournir un second corps de comprimé (202) comprenant une seconde poudre comprimée (200) ayant une seconde substance pharmacologiquement active ; et
une unité d'adhésion pour former le comprimé multicouche (450) en faisant adhérer le premier corps de comprimé (102) et le second corps de comprimé (202) ensemble par le biais d'un adhésif (308, 700),
**caractérisé en ce que** l'unité d'alimentation comprend une première bande transporteuse (1502) conçue pour transporter le premier corps de comprimé (102) vers une position d'adhésion (1504) et comprend une seconde bande transporteuse (1506) conçue pour transporter le second corps de comprimé (202) vers la position d'adhésion (1504), dans lequel la première bande transporteuse (1502) et la seconde bande transporteuse (1506) sont disposées de façon à converger dans l'espace au niveau de la position d'adhésion (1504), de sorte que le premier corps de comprimé (102) et le second corps de comprimé (202) soient comprimés ensemble par la convergence de la première bande transporteuse (1502) et de la seconde bande transporteuse (1506) dans la position d'adhésion (1504).

2. Appareil (1500) selon la revendication 1, dans lequel au moins un élément parmi la première bande transporteuse (1502) et la seconde bande transporteuse (1506) comprend une pluralité d'espaces de logement (1508) disposés le long d'une trajectoire de mouvement de la bande transporteuse respective, chacun étant conçu pour loger un corps de comprimé respectif.

3. Appareil (1500) selon la revendication 1 ou 2, dans lequel au moins un élément parmi la première bande transporteuse et la seconde bande transporteuse (1506) comprend au moins un rouleau d'entraînement (1512,1514) conçu pour entraîner la bande transporteuse respective.

4. Appareil (1500) selon l'une quelconque des revendications 1 à 3, dans lequel au moins un élément parmi la première bande transporteuse (1502) et la seconde bande transporteuse (1506) comprend au moins un rouleau de façonnage (1514) conçu pour définir une forme de la bande transporteuse respective.

5. Appareil (1500) selon l'une quelconque des revendications 1 à 4, dans lequel l'unité d'alimentation comprend un premier canal d'alimentation (1516) en communication avec la première bande transporteuse et conçu pour transporter le premier corps de comprimé (102) par le biais du premier canal d'alimentation (1516) à la première bande transporteuse (1502), et comprend un second canal d'alimentation (1518) en communication avec la seconde bande transporteuse (1506) et conçu pour transporter le second corps de comprimé (202) par le biais du second canal d'alimentation (1518) à la seconde bande transporteuse (1506).

6. Appareil (1500) selon la revendication 5, dans lequel les canaux d'alimentation sont disposés de façon à transporter le corps de comprimé respectif en direction de la bande transporteuse respective, entraîné par une force gravitationnelle.

7. Appareil (1500) selon l'une quelconque des revendications 1 à 6, dans lequel l'unité d'adhésion comprend un canal d'alimentation d'adhésif (1520), disposé de façon à transporter l'adhésif (308) en direction d'au moins un élément parmi le premier corps de comprimé (102) et le second corps de comprimé (202), entraîné par une force gravitationnelle.

8. Appareil (1500) selon l'une quelconque des revendications 5 à 7, comprenant une plaque de support (1522), dans lequel au moins une partie du groupe constitué du premier canal d'alimentation (1516), du second canal d'alimentation (1518) et du canal d'alimentation d'adhésif (1520) est formée d'un seul bloc dans la plaque de support (1522) et/ou dans lequel au moins une partie du groupe constitué de la première bande transporteuse (1502) et de la seconde bande transporteuse (1506) est montée sur la plaque de support (1522).

9. Appareil (1500) selon l'une quelconque des revendications 1 à 8, comprenant une unité de réglage de température (2304) conçue pour régler, en particulier au niveau de l'unité d'adhésion, une température d'au moins un élément dans le groupe constitué du premier corps de comprimé (102), du second corps de comprimé (202) et de l'adhésif (308).

10. Appareil (1500) selon la revendication 9, dans lequel l'unité de réglage de température (2304) est conçue pour chauffer au moins un élément dans le groupe constitué du premier corps de comprimé (102), du second corps de comprimé (202) et de l'adhésif (308) avant et/ou pendant l'adhésion.

11. Appareil (1500) selon l'une quelconque des revendications 9 à 10, dans lequel l'unité d'ajustement de température (2304) est disposée au niveau d'une partie de convergence dans l'espace entre la première bande transporteuse (1502) et la seconde bande transporteuse (1506).

12. Appareil (1500) selon l'une quelconque des revendications 9 à 11, dans lequel l'unité de réglage de température (2304) comprend au moins un support de refroidissement ayant au moins un canal de refroidissement et conçu pour qu'un agent de refroidissement puisse être conduit à travers l'au moins un canal de refroidissement.

13. Appareil (1500) selon l'une quelconque des revendications 1 à 12, dans lequel chacun des éléments parmi la première bande transporteuse (1502) et la seconde bande transporteuse (1506) a une section verticale (1510, 2512) et une section inclinée (2514, 2516) raccordée de manière angulaire, dans lequel les sections verticales (1510, 2512) sont disposées parallèlement et juxtaposées les unes aux autres, de façon à former un Y avec les sections inclinées (2514, 2516).

14. Appareil (1500) selon la revendication 13, dans lequel la section verticale (1510, 2512) d'au moins un élément parmi la première bande transporteuse (1502) et la seconde bande transporteuse (1506) a un mécanisme d'ajustement de pression conçu pour ajuster une pression selon laquelle le premier corps de comprimé (102) et le second corps de comprimé (202) sont comprimés ensemble pour former le comprimé multicouche (450).

15. Procédé de fabrication d'un comprimé multicouche (450) en utilisant un appareil (1500) selon l'une quelconque des revendications 1 à 14, ledit procédé comprenant :
la fourniture d'un premier corps de comprimé (102) comprenant une première poudre comprimée (100) ayant une première substance pharmacologiquement active ;
la fourniture d'un second corps de comprimé (202) comprenant une seconde poudre comprimée (200) ayant une seconde substance pharmacologiquement active ;
la formation du comprimé multicouche (450) en faisant adhérer le premier corps de comprimé (102) et le second corps de comprimé ensemble par le biais d'un adhésif (308,700).
